# EUROPÄISCHE PATENTSCHRIFT

(11) **EP 2 090 329 B1**
(45) Veröffentlichungstag und Bekanntmachung des Hinweises auf die Patenterteilung: **23.01.2013**
(21) Anmeldenummer: 09150824.2
(22) Anmeldetag: 19.01.2009
(51) Int. Cl.: A61M 25/10

(54) **Katheter, System zum Einbringen einer intraluminalen Endoprothese sowie Verfahren zur Herstellung derselben**
Catheter, system for applying an intraluminal endoprosthetis and method for producing same
Cathéter, système d'introduction d'une endoprothèse intraluminale et procédé de fabrication de celle-ci

(30) Priorität: 13.02.2008 DE 102008008925
(43) Veröffentlichungstag der Anmeldung: 19.08.2009
(73) Patentinhaber: Biotronik VI Patent AG, 6341 Baar (CH)
(72) Erfinder: Wesselmann, Matthias, 8912, Glattfelden (CH); Angst, Markus, 8047, Zürich (CH)
(74) Vertreter: Lindner-Vogt, Karin L.

(56) Entgegenhaltungen:
- EP-A- 0 414 350
- EP-A- 1 738 791
- WO-A-01/85229
- WO-A-03/049603
- US-A- 5 041 125
- US-A1- 2007 129 748
- US-A1- 2007 244 501

## Beschreibung

Die vorliegende Erfindung bezieht sich auf einen Katheter mit einem Ballon, wobei der Ballon in einem nicht dilatierten Zustand mindestens einen Flügel aufweist. Einen solchen Katheter beschreibt US-A-2007/0244501, das als nächtligender Stand der Technik anzusehen ist. Die Erfindung bezieht sich ferner auf ein System zum Einbringen einer intraluminalen Endoprothese, vorzugsweise eines Stents, in einen Körperhohlraum bestehend aus der intraluminalen Endoprothese und einem derartigen Katheter sowie einem Verfahren zur Herstellung eines derartigen Katheters und einem Verfahren zur Herstellung eines derartigen Systems.

Katheter sind Röhrchen oder Schläuche verschiedenen Durchmessers, die in den jeweiligen zu behandelnden Körperhohlraum eingeführt werden können. Sogenannte Ballonkatheter, die vor allem in der Angioplastie zur Erweiterung oder Wiedereröffnung eines Gefäßes eingesetzt werden, weisen einen Führungsdraht auf, der zuerst in das zu behandelnde Gefäß eingeschoben wird. Anschließend wird ein Schlauch, der in einem vorgegebenen Bereich des Schlauchs einen nicht dilatierten, gefalteten Ballon aufweist, entlang des Führungsdrahts bis zu der zu behandelnden Stelle des Gefäßes vorgeschoben, so dass der Ballon im Bereich der zu behandelnden Stelle des Gefäßes, die z.B. eine Stenose aufweist, platziert ist. Danach wird der Ballon dilatiert, d.h. entfaltet und aufgedehnt, so dass die zu behandelnde Stelle wiedereröffnet oder erweitert wird und der Strom der Körperflüssigkeit in dem Gefäß nicht mehr oder nicht mehr in dem zuvor vorhandenen Maße behindert wird. Schließlich wird der Ballon wieder entleert und entlang des Führungsdrahts wieder aus dem Gefäß entfernt. Gleichzeitig oder anschließend wird auch der Führungsdraht aus dem Gefäß zurückgezogen.

Zum Einführen von Ballonkathetern ist es notwendig, dass der Ballon zunächst im gefalteten, d.h. nicht dilatierten, Zustand in den zu behandelnden Körperhohlraum eingebracht wird. Bei derzeit verwendeten Ballonkathetern werden die Ballone nach dem Formen des Ballons in Falten gelegt und anschließend durch Aufbringen von Druckkräften auf den gefalteten Ballon (Prägen) fixiert. Die hierfür verwendeten Arbeitsschritte sowie die entsprechende Vorrichtung sind aufwendig, da im Prägeschritt hohe Temperaturen und lange Prägezeiten erforderlich sind. Zudem bedingt die Wärmebehandlung häufig ein Schrumpfen des Ballons, so dass die Maße des Ballons verändert werden. Ein weiterer Nachteil dieses Vorgehens besteht darin, dass die Flügelform nach dem ersten Dilatieren verloren geht, so dass sich der Ballon beim Deflatieren anders als zuvor und zum Teil fehlerhaft zurückfaltet. Dies wird dadurch verursacht, dass eine auf herkömmlichem Weg hergestellte Falte beim Aufblasen des Ballons nur wenig Energie speichert, so dass die Rückfaltneigung beim Deflatieren gering ist. Beim Zurückziehen eines fehlerhaft zurückgefalteten Ballons wird eine erhöhte Pullbackkraft benötigt. Ebenso wird eine erhöhte Kraft benötigt, um einen Stent nachzudilatieren, einen Seitenast durch ein Stentsegment zu erreichen oder um eine 2. Stenose zu passieren.

Bei herkömmlichen Kathetern weist der Ballon Flügel auf, die parallel zu Achse des Ballons verlaufen. Diese Flügel erzeugen ein anisotropes Biegemoment des gefalteten Ballons. Der Nachteil einer derartigen Faltung besteht darin, dass der Katheter im Bereich des Ballons anfälliger für ein Knicken des Katheters wird und die Flügel sich in engen Kurven beim Einführen in den zu behandelnden Körperhohlraum aufstellen, so dass das Einführen behindert wird. Nach dem Deflatieren können außerdem Flügel, die parallel zur Katheterachse verlaufen, unerwünschte Querfalten verursachen, wenn der Innenschaft sich nicht plastisch mit dem Ballon dehnt. Auch hierdurch kann eine erhöhte Pullbackkraft benötigt werden.

Ferner können integral mit einer pharmazeutisch aktiven Substanz versehene Ballone bei unkontrolliertem Rückfalten zum falschen Zeitpunkt, beispielsweise während der Rückführung, Restmengen der Substanz an die umgebende Körperflüssigkeit abgeben. Dies kann unerwünschte Nebenwirkungen auslösen.

Unter einer "pharmazeutisch aktiven Substanz" (oder therapeutisch aktive oder wirksame Substanz) im Sinne der Erfindung wird ein pflanzlicher, tierischer oder synthetischer Wirkstoff (Medikament) oder ein Hormon verstanden, das in geeigneter Dosierung als Therapeutika zur Beeinflussung von Zuständen oder Funktionen des Körpers, als Ersatz für natürlich vom menschlichen oder tierischen Körper erzeugte Wirkstoffe, wie Insulin, sowie zur Beseitigung oder zum Unschädlichmachen von Krankheitserregern, Tumoren, Krebszellen oder Körperfremdstoffen Einsatz findet. Die Freisetzung der Substanz in der Endoprothesenumgebung hat einen positiven Effekt auf den Heilungsverlauf oder wirkt pathologischen Veränderungen des Gewebes in Folge des chirurgischen Eingriffs entgegen bzw. dient dem Unschädlichmachen von maladen Zellen in der Onkologie.

Derartige pharmazeutisch aktive Substanzen weisen beispielsweise eine antiinflammatorische und/oder antiproliferative und/oder spasmolytische Wirkung auf, wodurch beispielsweise Restenosen, Entzündungen oder (Gefäß-)Spasmen vermieden werden können. Derartige Substanzen können in besonders bevorzugten Ausführungsbeispielen aus einer oder mehreren Substanzen der Wirkstoffgruppe der Calciumkanalblocker, der Lipidregulatoren (wie beispielsweise Fibrate), der Immunsuppressiva, der Calcineurininhibitoren (wie beispielsweise Tacrolimus), der Antiflogistika (wie beispielsweise Cortison oder Dichlofenac), der Antiinflammatorica (wie beispielsweise Imidazole), der Antiallergika, der Oligonucleotide (wie beispielsweise dODN), der Östrogene (wie beispielsweise Genistein), der Endothelbildner (wie beispielsweise Fibrin), der Steroide, der Proteine, der Hormone, der Insuline, der Zytostatika, der Peptide, der Vasodilatatoren (wie beispielsweise Sartane) und der antiproliferativ wirkenden Stoffe, der Taxole oder Taxane, hier vorzugsweise Paclitaxel oder Sirolimus, bestehen.

In der Druckschrift DE 691 19 753 T2 wird ein Ballonkatheter beschrieben, der einen Katheterkörper und einen Ballon aufweist, der entlang der Länge des Katheterkörpers angeordnet ist. Der Ballon ist ferner mit einer Einrichtung zu seinem Aufblasen und Ablassen von außen und einer Einrichtung zum Zuführen eines Medikaments oder einer Kombination von Medikamenten zur Behandlung oder Diagnose innerhalb eines Körperhohlorgans versehen, wenn der Katheter in diesem positioniert und aufgeblasen wird. Die Zuführeinrichtung weist Mikrokapseln an der Außenseite des Ballons auf, wobei die Mikrokapseln in Falzen am Ballon, wie sie ausgebildet sind, wenn der Ballon geschrumpft ist, an ihm festgehalten werden. Hierbei sind die Mikrokapseln derart gestaltet, dass sie zerreißbar oder abbaubar sind. Sie öffnen sich, wenn sie an den Hohlorganwänden zurückgelassen werden. Die Kapseln können auch aufgrund des Anlegens von Ultraschallwellen zerreißen.

Der Nachteil des in der oben angegebenen Druckschrift beschriebenen Katheters besteht darin, dass zur Realisierung der Medikamentenabgabe mikroverkapselte Medikamente verwendet werden müssen, die in ihrer Herstellung teuer und aufwendig sind. Eine Mikroverkapselung ist zudem nicht für jedes in Frage kommende Medikament herstellbar. Außerdem weist ein Ballon eines derartigen, mit Mikrokapseln versehenen Katheters einen vergleichsweise großen Durchmesser auf, dessen Profil in der Praxis nicht verwendbar ist und den Katheter steif und unflexibel macht. Zudem kann das Medikament nicht nur an der zu behandelnden Stelle sondern auch beim Einführen oder der Dilatation des Ballons abgerieben werden. Hierdurch werden die mit der Behandlung verbundenen Nebenwirkungen erhöht.

Ballonkatheter können auch dazu verwendet werden, intraluminale Endoprothesen an eine zu behandelnde Stelle in einem Körperhohlraum einzubringen.

Intraluminale Endoprothesen in Form von Stents werden derzeit weit verbreitet eingesetzt, da sie eine einfache und kostengünstige Behandlung ermöglichen. Sie weisen häufig ein rohrförmiges oder hohlzylinderförmiges Grundgitter auf, das an beiden Längsenden offen ist. Das Grundgitter einer derartigen Endoprothese wird mittels eines Katheters in den zu behandelnden Körperhohlraum eingesetzt und dilatiert oder freigesetzt. Nach Entfernen des Katheters dient die Endoprothese dazu, den Körperhohlraum zu stützen. Derartige Stents haben sich insbesondere zur Behandlung von Gefäßerkrankungen etabliert. Durch den Einsatz von Stents können verengte Bereiche in den Gefäßen so erweitert werden, dass das Gefäßlumen vergrößert wird.

Intraluminale Endoprothesen werden heute häufig ebenfalls mit pharmazeutisch aktiven Substanzen versehen, die über einen bestimmten Zeitraum im Organismus freigesetzt werden.

Diese pharmazeutisch aktiven Substanzen können beispielsweise dazu dienen, Restenosen oder Agglomerationen zu vermeiden. Durch die Freisetzung von pharmazeutisch aktiven Substanzen, mit denen derartige intraluminale Endoprothesen versehen sind, ist es möglich, lediglich eine Iokale Behandlung, d.h. eine Elution eines Wirkstoffs im Wesentlichen nur in das die intraluminale Endoprothese umgebende Gewebe, vorzunehmen. Dieser Vorgang wird auch als "Local Drug Delivery" (LDD) bezeichnet. Der Behandlungsort, an dem der Wirkstoff seine pharmakologische Wirkung entfalten soll, grenzt somit unmittelbar an den Ort der Implantation der intraluminalen Endoprothese.

Derzeit werden auch intraluminale Endoprothesen eingesetzt, die aus einem Material bestehen, das der Biodegradation unterliegt. Hierbei werden unter Biodegradation hydrolytische, enzymatische oder andere stoffwechsel-bedingte Abbauprozesse im lebenden Organismus verstanden, die vor allem durch die mit der Endoprothese in Kontakt gelangenden Körperflüssigkeiten verursacht werden und zu einer allmählichen Auflösung zumindest großer Teile der Endoprothese führen. Synonym zu dem Begriff Biodegradation wird häufig der Begriff Biokorrosion verwendet. Der Begriff Bioresorption umfasst die anschließende Resorption der Abbauprodukte durch den lebenden Organismus. Derartige biodegradierbare Materialien können aus Polymeren oder Metallen realisiert werden. Im Zusammenhang mit Stents ist auch die Abkürzung AMS (Absorbierbarer Metall Stent) gebräuchlich. Derartige Stents enthalten ein biodegradierbares Metall, vorzugsweise Magnesium und/oder eine Magnesiumlegierung. Weitere in Frage kommende degradierbare Metalle sind Eisen, Zink, Wolfram und deren Legierungen.

Bei intraluminalen Endoprothesen, die aus einem biodegradierbaren Material bestehen und außerdem mit einer pharmazeutisch aktiven Substanz versehen sind, entsteht häufig das Problem, dass die pharmazeutisch aktive Substanzen nicht richtig am Grundgitter der Endoprothese haften oder nicht in der gewünschten Weise funktionieren, da sich bei der Biodegradation der Endoprothese der pH-Wert der Umgebung verändern und/oder die Endoprothese unkontrolliert korrodieren kann und dabei stark unterwandert wird. Die Abgabe der pharmazeutisch aktiven Substanz erfolgt somit nicht in der gewünschten Weise oder dem gewünschten Zeitraum.

Die Aufgabe der vorliegenden Erfindung besteht darin, ein Katheter zu schaffen, der sich einfacher falten lässt und vor allem beim Zurückfalten die obigen Probleme des Katheters vermeidet. Die Aufgabe besteht ferner darin, ein System aus einem Katheter und einer intraluminalen Endoprothese zu schaffen, das zusätzlich die Freisetzung der pharmazeutisch aktiven Substanz beim Einbringen der intraluminalen Endoprothese auf den Ort beschränkt, an dem die intraluminale Endoprothese eingesetzt wird.

Die Aufgabe besteht ferner darin, ein Verfahren zur Herstellung eines derartigen Katheters und eines derartigen Systems anzugeben, das einfach und kostengünstig ist.

Die obige Aufgabe wird durch einen Katheter gelöst, an und/oder in dessen Ballonmaterial für jeden Flügel mindestens ein im Wesentlichen in Längsrichtung verlaufendes Faltelement vorgesehen ist, das beim Falten des Ballons in einem der in Längsrichtung verlaufenden Bereiche des Flügels mit einem Minimum im Biegeradius angeordnet ist.

Hierbei ist unter der Längsrichtung die Richtung der Achse des Katheters zu verstehen. Mit dem "im Wesentlichen in Längsrichtung verlaufenden Faltelement" ist ein Faltelement gemeint, das überwiegend in Längsrichtung verläuft, d.h. auch schräg oder spiralig in Längsrichtung, also mit einer Komponente in eine Richtung senkrecht zur Längsrichtung, verlaufen kann. Falten oder Faltlinien sind Bereiche der Ballonmembran, in denen diese ein Minimum im Biegeradius aufweisen. Diese Falten entstehen, wenn die Ballonmembran außen (am äußeren Flügelende) oder innen (an der Verbindungsstelle benachbarter Flügel) überdehnt wird. Unter dem Begriff "Falten" im Zusammenhang mit "Falten des Ballons" wird sowohl das erstmalige Falten des Ballons bei der Herstellung des Katheters als auch das Zurückfalten (Rewrap) beim Deflatieren verstanden.

Die erfindungsgemäße Gestaltung des Ballons mit dem mindestens einen Faltelement hat den Vorteil, dass der Ballon entlang des mindestens einen Faltelements gefaltet wird, so dass die vorbestimmte und gewünschte Anordnung der Flügel am Ballon erleichtert wird. Insbesondere kollabiert der Ballon beim Rückfalten und bei weiteren Faltungen des Ballons immer wieder entlang des mindestens einen Faltelements, so dass das Zurückfalten (Rewrap) reproduzierbar wird. Hierdurch werden fehlerhafte Rückfaltungen und erhöhte Pullbackkräfte vermieden.

Hierbei ist es besonders bevorzugt, wenn das mindestens eine Faltelement verglichen mit den übrigen Bereichen des Ballons eine veränderte Steifigkeit, vorzugsweise eine niedrigere Steifigkeit, aufweist. Dadurch wird das Falten entlang der Verlaufsrichtung des Faltelements erleichtert, da die Ballonmembran immer entlang der schwächsten Stelle einknickt, sobald der Ballon entleert wird.

Eine bevorzugte Möglichkeit, Steifigkeitsunterschiede in die Ballonmembran zu integrieren, besteht darin, dass der Ballon im Bereich des mindestens einen Faltelements Vertiefungen oder Erhebungen oder mindestens einen Wandstärkensprung aufweist.

Alternativ oder zusätzlich zu den oben genannten Möglichkeiten, Faltelemente, vorzugsweise Steifigkeitsunterschiede, in den Ballon zu integrieren, besteht außerdem die ebenfalls vorteilhafte Möglichkeit, dass das mindestens eine Faltelement einen Bereich des Ballons ausbildet, der eine von den übrigen Bereichen des Ballons verschiedene Materialzusammensetzung aufweist. Diese Bereiche (mit der größten Ausdehnung in Längsrichtung des Ballons verlaufend und bspw. mit einem quadratischen, kreisförmigen, ellipsenförmigen oder rechteckigen Querschnitt aufweisend) sind bspw. stegförmige ausgebildet, wobei ein solcher Steg auf der Oberfläche des Ballons oder eingebettet in das Volumen des Ballons vorgesehen werden können. Beispielsweise können bei einem Ballonmaterial aus PEBAX in Längsrichtung auf der Außenseite entlang des Ballons verlaufende Bereiche mit dem Material PA12 eingebracht werden, die beim Falten unter stärkerem Zug als das Ballonmaterial stehen. Beim Falten des Ballons werden sich diese Bereiche somit in den Minima des Biegeradius anordnen, bei denen ein Flügel des Ballons an den benachbarten Flügel angrenzt. Bei der Verwendung von PA12 als Ballonmaterial können umgekehrt in Längsrichtung auf der Innenseite entlang des Ballons verlaufende Bereiche des Materials PEBAX eingebracht werden. Diese Bereiche werden sich beim Falten des Ballons an der Spitze eines Flügels anordnen, da diese Bereiche unter geringerer Zugspannung als das übrige Ballonmaterial stehen. Beide Varianten können auch kombiniert werden. Weitere Materialkombinationen können außerdem die Materialien PA11 und PVC nutzen. In einem weiteren Ausführungsbeispiel, bei dem ebenfalls die genannten Materialien verwendet werden können, wird das nicht zu den Faltelementen gehörende Material des Ballons mit einer Verstärkungsschicht versehen werden, die Bereiche erhöhter Wandstärke erzeugt. Die Verstärkungsschicht kann als Zwischenschicht in das Material des Ballons eingebettet sein.

In einem weiteren bevorzugten Ausführungsbeispiel weist das mindestens eine Faltelement, das vorzugsweise als Vertiefung oder Erhebung ausgebildet ist, Unterbrechungen auf, die für eine größere Stabilität der Faltlinien sorgen.

Ebenfalls bevorzugt ist ein Katheter, bei dem das mindestens eine Faltelement in einem fest vorgegebenen Winkel zur Ballonachse verläuft. Dies bedeutet auch, dass der jeweilige Flügel in einem Winkel zur Ballonachse erzeugt wird. Hierbei verläuft das mindestens eine Faltelement auf oder in der Fläche des Ballons um den Ballon herum und nicht lediglich parallel zur Ballonachse. Hierdurch wird ein gleichmäßiges Biegemoment des gefalteten Ballons um seine Längsachse erzeugt. Bei einer von außen aufgebrachten Biegung des Systems heben sich Zugkräfte und Kompressionen weitgehend auf, Dehnungen und Streckungen kompensieren sich. Der erfindungsgemäße Katheter zeichnet sich durch eine gleichmäßige Trackability aufgrund des winkelunabhängigen Biegemoments des gefalteten Ballons aus. Ferner stellen sich die umlaufenden Flügel in engen Kurven nicht mehr auf, da sich die herrschenden Kräfte, wie oben beschrieben, ausgleichen und der steife Flügel nicht mehr senkrecht zur Beanspruchungsrichtung steht und damit Deformationen an ihrer Flanke besser abbauen kann.

Bei einem weiteren Ausführungsbeispiel eines erfindungsgemäßen Katheters werden die Faltelemente des Ballons durch Längsstreben ausgebildet, die ein Gerüst bilden, welches an der Innenseite und/oder an der Außenseite des Ballons angeordnet ist und das den Ballon an definierten Stellen stützt. Zudem lassen sich mit dem Gerüst je nach Wahl des Gerüstmaterials sehr große Spannungen beim Falten des Ballons aufnehmen. Die Längsstreben können auch spiralig verlaufen. Als Gerüstmaterialien können beispielsweise Nitinol oder Thermoplaste verwendet werden.

In einem ebenfalls bevorzugten Ausführungsbeispiel weist der Katheter im nicht dilatierten Zustand mindestens eine pharmazeutisch aktive Substanz auf, die zumindest teilweise unter dem mindestens einen Flügel des Ballons des Katheters angeordnet ist.

Hierbei beinhaltet der "nicht dilatierte Zustand" alle Zustände des Ballons des Katheters, in dem dieser nicht vollständig entfaltet vorliegt, d.h. noch mindestens einen Flügel an dem Ballon mindestens teilweise vorhanden ist, auch wenn dieser bereits teilweise aufgedehnt ist. Ebenso werden Zustände mit einbezogen, bei denen der Ballon deflatiert ist, d.h. bereits einmal dilatiert war und anschließend wieder zusammengefaltet wurde. Mit dem "nicht dilatierten Zustand" werden aber insbesondere Zustände angesprochen, in denen der Ballon nach dem Falten und Prägen vollständig gefaltet vorliegt bzw. nach dem Deflatieren wieder fast vollständig eingefaltet ist.

Außerdem bedeutet "unter dem mindestens einen Flügel des Ballons angeordnet", dass die mindestens eine pharmazeutisch aktive Substanz innerhalb des Flügels des Ballons, d.h. auf oder in den Oberflächen der Flügel angeordnet ist, die nach dem Falten übereinander liegen. Die unter dem Flügel angeordnete pharmazeutisch aktive Substanz wird somit von dem außen liegenden Teil des jeweiligen Flügels abgedeckt.

Bei der Anordnung einer pharmazeutisch aktiven Substanz unter dem mindestens einen Flügel ist es bevorzugt, wenn die mindestens eine pharmazeutisch aktive Substanz auf einem Träger oder mehreren Trägern vorgesehen ist, die unter einem Flügel angeordnet ist oder mehreren Flügeln des Ballons angeordnet sind. Hierdurch ist es in besonders einfacher Weise möglich, einen bereits einmal verwendeten Katheter wieder zur Abgabe einer pharmazeutisch aktiven Substanz einzusetzen, da ein oder mehrere Träger leicht unter dem Flügel oder den Flügeln angeordnet werden können.

Besonders bevorzugt ist es außerdem, wenn die Flügel des Ballons mittels der mindestens einen pharmazeutisch aktiven Substanz verklebt sind. Hierdurch verstärkt sich der Effekt, dass die pharmazeutisch aktive Substanz aus den Flügeln nicht unkontrolliert entweicht. Die Klebverbindung ist dabei allerdings so beschaffen, dass bei der Dilatation des Ballons diese aufgebrochen wird, so dass der Ballon ohne bedeutende Verzögerung und ohne größeren Kraftaufwand dilatiert werden kann.

Besonders kostengünstig herstellbar ist der Katheter, wenn die mindestens eine pharmazeutisch aktive Substanz, vorzugsweise enthaltend Taxole und/oder Taxane, besonders bevorzugt Paclitaxel oder Sirolimus, und/oder vorzugsweise enthaltend mindestens einen hyperplastischen Wirkstoff mit einem Verteilungskoeffizienten zwischen Butanol und Wasser von ≥ 0,5, mittels Tauchen, Sprühen, Bestreichen oder Pressen auf dem Ballon, ggf. eingebettet in einen Träger oder Carrier, aufgebracht ist. Hierbei dient der ggf. in der pharmazeutisch aktiven Substanz enthaltene hyperplastische Wirkstoff zur örtlich begrenzten Behandlung potentiell hyperproliferativen Gewebes. Als antihyperplastischer Wirkstoff können beispielsweise ein Cytostatikum, ein Kortikoid, ein Prostacyclin, ein Antioxidans, ein Mittel zur Hemmung der Zellproliferation oder ein Immunsuppressivum verwendet werden.

Die obige Aufgabe wird ferner durch ein System gelöst, bei dem auf einem oben dargestellten, erfindungsgemäßen Katheter die intraluminale Endoprothese auf dem gefalteten Ballon derart fest angeordnet ist, dass sie diesen mindestens teilweise umgibt.

Hierbei wird durch die Angabe, dass die Endoprothese den gefalteten Ballon mindestens teilweise umgibt, ausgedrückt, dass die Endoprothese außen auf den nach der Faltung außen liegenden Flächen des Ballons angeordnet ist. Die Endoprothese bedeckt diese außen liegenden Flächen dabei mindestens teilweise. Hierbei ist die Anordnung der Endoprothese auf dem Ballon nicht als dauerhaft fest anzusehen. Die Endoprothese ist bis zum Beginn der Ballondilatation fest auf dem Ballon angeordnet, also auch bei der Lagerung und beim Einführen in den menschlichen oder tierischen Körper. Nach dem Erreichen des maximal dilatierten Zustandes, wenn der Ballon wieder entleert wird, verbleibt die Endoprothese im Körperhohlraum, während der Katheter aus diesem entfernt wird.

Durch das oben angegebene System kann die nicht notwendiger Weise mikroverkapselt vorliegende pharmazeutisch aktive Substanz direkt dort, wo die intraluminale Endoprothese in den Körperhohlraum implantiert wird, an die umgebende Körperflüssigkeit und das Gewebe abgegeben werden, da erst durch das Dilatieren die pharmazeutisch aktive Substanz durch Öffnen der Flügel freigegeben wird. Zudem ist durch die feste Anordnung der intraluminalen Endoprothese auf dem Ballon gewährleistet, dass das System aus intraluminaler Endoprothese und Ballon beim Einführen des Systems in den Körperhohlraum ein insgesamt sehr kleines Volumen einnimmt, so dass es flexibel und einfach handhabbar ist. Es ist außerdem nicht notwendig, die pharmazeutisch aktive Substanz in einer Mikroverkapselung bereitzustellen. Zudem kann die Freigabe der mindestens einen pharmazeutisch aktiven Substanz aufgrund des Fehlens der Mikroverkapselung sofort nach der Dilatation des Ballons zusammen mit der intraluminalen Endoprothese erfolgen, so dass sich eine schnelle Wirksamkeit der pharmazeutisch aktiven Substanz und eine Wirksamkeit genau am Ort der Behandlung ergibt.

In einem besonders bevorzugten Ausführungsbeispiel ist bei dem erfindungsgemäßen System die intraluminale Endoprothese auf den Ballon aufgecrimpt. Dies ist eine sehr einfache und kostengünstige Anbringungsweise der intraluminalen Endoprothese auf dem Ballon.

Ferner ist es von Vorteil, wenn die intraluminale Endoprothese als biodegradierbarer Stent, vorzugsweise als AMS-Stent, ausgebildet ist. Ein derartiger Stent ist nach Erfüllung seiner Behandlungsfunktion nicht länger im Gewebe des behandelten Körperhohlraums vorhanden (löst sich nahezu vollständig auf) und verursacht so weniger Nebenwirkungen. Da die pharmazeutisch aktive Substanz nur während der kurzen Dilatationszeit des Ballons des Katheters an die Umgebung abgegeben wird, beeinflusst die Degradation der biodegradierbaren Endoprothese nicht oder nur sehr gering die Wirkung der pharmazeutisch aktiven Substanzen, da zu diesem Zeitpunkt die Degradation noch nicht oder nur in unbedeutendem Maße begonnen hat.

Eine besonders gute Abdeckung des mit der pharmazeutisch aktiven Substanz versehenen Ballons im nicht dilatierten Zustand wird dann erreicht, wenn in einem bevorzugten Ausführungsbeispiel die intraluminale Endoprothese die darunter liegenden äußeren Flächen des gefalteten Ballons vollständig bedeckt. Hierbei werden mit "äußeren Flächen" die Außenflächen oder Oberflächenbereiche des gefalteten Ballons gemeint, die nach dem Falten und Prägen außen liegen. Die unterhalb des Flügels oder der Flügel angeordneten, übereinander liegenden Außenflächen oder Oberflächenbereiche des Ballons gehören nicht zu den äußeren Flächen.

In einem besonders bevorzugten Ausführungsbeispiel ist die intraluminale Endoprothese luminal mit einer leicht lösbaren Beschichtung versehen, vorzugsweise mit einer oder mehrerer der Substanzen aus der Gruppe Zucker, vorzugsweise Polysaccharide, Glykane, Glucose, Glycogen, Amylose, Amylopektin, Chitin, Callose und Zellulose, und Fette, vorzugsweise Cholesterin, Cholesterol, Palmöl, partiell hydrierte Sojaöle und gesättigte Öle. Eine derartige Schicht wird nach der Implantation durch die Körperflüssigkeit leicht abgewaschen, so dass keine Wirkstoffreste an der luminalen Seite der intraluminalen Endoprothese verbleiben und somit die Endothelialisierung nicht behindert wird.

Die obige Aufgabe wird außerdem durch ein Verfahren zur Herstellung eines Katheters gelöst, bei dem der Ballon zunächst mit mindestens einem Faltelement versehen wird, danach der Kathetergrundkörper mit Innenschaft und Außenschaft bereitgestellt und anschließend der Ballon mit dem Innenschaft und dem Außenschaft verbunden wird. Hierbei weist der Kathetergrundkörper einen Innenschaft und einen Außenschaft auf, wobei der Außenschaft den Innenschaft umgibt. Der Ballon ist an einem Ende mit dem Innenschaft und dem anderen Ende mit dem Außenschaft verbunden. Ggf. sind im Bereich des Ballons auf dem Innenschaft Markierungen angeordnet, die z.B. als Röntgenmarker dienen. Das erfindungsgemäße Verfahren zur Herstellung eines Katheters lässt sich einfach realisieren und ist kostengünstig in der Durchführung.

In einem weiteren Ausführungsbeispiel kann der Ballon auch zunächst ohne ein Faltelement mit dem bereitgestellten Kathetergrundkörper verbunden werden, wobei hierfür der Ballon an einem Ende mit dem Innenschaft und an dem anderen Ende mit dem Außenschaft verbunden wird. Anschließend wird in den Ballon das mindestens eine Faltelement eingebracht, wobei dieses z.B. mittels chemischer Verfahren erzeugt wird.

Bei beiden Verfahren folgt bei der Herstellung des Katheters das Falten des Ballons und das Prägen entsprechend der vorgelegten Falten des Ballons.

Ein vorteilhaftes Herstellungsverfahren eines erfindungsgemäßen Katheters beinhaltet die Herstellung des Ballons vor der Verbindung mit dem Kathetergrundkörper mittels Blasformen, wobei die Blasform dort, wo das mindestens eine Faltelement entstehen soll, mit einer Vertiefung oder Erhebung oder mehreren Vertiefungen oder Erhebungen, beispielsweise in Form von runden oder länglichen Noppen oder unterbrochenen Rillen, versehen ist. Alternativ kann der Ballon mittels Spritzblasen hergestellt werden, wobei im Bereich des mindestens einen Faltelements mindestens ein definierter Wandstärkesprung erzeugt wird. Sowohl durch die Vertiefungen und Erhebungen der Blasform als auch durch den mindestens einen Wandstärkesprung beim Spritzblasen werden auf einfache Weise Steifigkeitsunterschiede im Ballon erzeugt, die zur Ausbildung des mindestens einen Faltelements führen.

Wie oben bereits erläutert, ist es außerdem vorteilhaft, den Ballon außen und/oder innen mit einem aus Längsstreben bestehenden Self-X-Gerüst zu versehen, welches den Ballon in bestimmten Bereichen stützt. Das Stützgerüst kann aus einem hochelastischen Material, das auch im maximal dilatierten Zustand (Rated Burst Pressure) immer noch an der Balloninnenwand anliegen muss, hergestellt werden. Wird der Balloninnendruck gesenkt, dann wird der Ballon nur noch im Bereich des Stützgerüsts offen gehalten und kollabiert in den nicht gestützten Bereichen zwischen den Längsstreben. So kann der Verlauf der Falten sehr genau gesteuert werden. Ein derartiges Gerüst könnte bspw. aus zwei spiralförmig verlaufenden Nitinoldrähten bestehen, die in den Ballonhälsen an Ringen verschweißt sind.

Das mindestens eine Faltelement kann auch derart vorteilhaft erzeugt werden, dass der Ballon im Bereich des mindestens einen Faltelements, vorzugsweise vor dem Aufbringen der mindestens einen pharmazeutisch aktiven Substanz, lokal thermisch, beispielsweise mittels eines Lasers, und/oder mittels eines Lösungsmittels behandelt wird. Außerdem kann der Ballon, ebenfalls vorzugsweise vor dem Aufbringen der mindestens einen pharmazeutisch aktiven Substanz, im Bereich außerhalb des mindestens einen Faltelements mit einem zusätzlichen verstärkenden Material versehen werden.

Wie oben bereits erläutert, ist es von Vorteil, wenn das mindestens eine Faltelement derart vorgesehen wird, dass es unter einem fest vorgegebenen Winkel zur Ballonachse auf der Oberfläche des Ballons oder in der Ballonoberfläche verläuft.

In einem bevorzugten Ausführungsbeispiel des Herstellungsverfahrens wird auf den Ballon des Katheters vor dem Falten zunächst mindestens seine pharmazeutisch aktive Substanz auf oder in die äußere Oberfläche des Ballons aufgebracht, vorzugsweise mittels Tauchen, Sprühen, Bestreichen oder Pressen. Ein derartiges Verfahren stellt besonders einfach und kostengünstig eine pharmazeutisch aktive Substanz auf oder in der Ballonoberfläche zur direkten Behandlung des Körperhohlraums, in den der Katheter eingeführt wird, bereit.

Besonders bevorzugt wird die pharmazeutisch aktive Substanz eingebettet in einen Träger auf den Ballon aufgebracht, wobei in dem Träger ggf. ein Kontrastmittel und/oder ein anorganisches Salz und/oder ein organisches Salz und/oder mindestens ein weiterer Zusatzstoff vorgesehen sein kann, der beispielsweise zur Verbesserung der mechanischen Haftung auf der Ballonoberfläche und/oder zur Verbesserung der Abgabe der pharmazeutisch aktiven Substanz an die Gefäßwand und/oder zur Verbesserung der Aufnahmefähigkeit der Gefäßwand dient. Die pharmazeutisch aktive Substanz enthält vorzugsweise Taxole und/oder Taxane, besonders bevorzugt Paclitaxel oder Sirolimus, und/oder vorzugsweise mindestens einen hyperplastischen Wirkstoff mit einem Verteilungskoeffizienten zwischen Butanol und Wasser von ≥ 0,5. Diese Zusatzstoffe verbessern die Eigenschaften des Trägers mit der pharmazeutisch aktiven Substanz.

Es ist weiterhin ein Herstellungsverfahren für einen Katheter bevorzugt, bei dem die überschüssige mindestens eine pharmazeutische aktive Substanz auf den nach dem Falten außen liegenden äußeren Flächen oder Oberflächen des Ballons nach dem Faltschritt entfernt wird, vorzugsweise durch Wegwischen. Hierdurch wird gewährleistet, dass die mindestens eine pharmazeutisch aktive Substanz lediglich unter dem Flügel angeordnet wird. Zum Wegwischen der pharmazeutisch aktiven Substanz kann beispielsweise poröses Zellpapier, Schwamm oder Ähnliches, gegebenenfalls getränkt mit einem Lösungsmittel, verwendet werden.

Als besonders vorteilhaft hat sich außerdem erwiesen, dass die mindestens eine pharmazeutisch aktive Substanz vor der Anordnung der intraluminalen Endoprothese auf dem Ballon ausgehärtet oder polymerisiert wird. Eine Aushärtung kann auch mit Hilfe eines Polymers oder eines Lösungsmittels auf der Ballonoberfläche erfolgen. Die pharmazeutisch aktive Substanz haftet hierdurch besonders gut an der Oberfläche des Ballons. Das Aushärten oder Polymerisieren wird insbesondere durch eine UV-Bestrahlung, eine Bestrahlung mittels Betastrahlen und/oder eine thermische Behandlung realisiert.

Die mindestens eine pharmazeutisch aktive Substanz wird vorzugsweise zusammen mit einem Träger oder Carrier (z.B. einem Polymer und/oder einem Lösungsmittel) aufgetragen. Im Fall der Verwendung eines Lösungsmittel verdampft dieses beim und/oder nach dem Auftragen. Das Austreiben des Lösungsmittels bei gleichzeitigem Aushärten des Polymer-Carriers wird auch als Curen bezeichnet. Als Lösungsmittel kommen, abhängig von der pharmazeutisch aktive Substanz, vorzugsweise folgende Stoffe in Betracht: DMSO, Aceton, Ether (Di-ethylether), Methanol, Isopropanol, Ester sowie weitere geeignete Alkohole. Beim Einsatz von Polymeren oder einer polymerähnlichen Substanz als Träger und Aushärthilfe, muss darauf geachtet werden, dass die Polymere oder die polymerähnliche Substanz leicht löslich sind oder das Medikament schnell abgeben. Unter diesem Gesichtspunkt eignen sich besonders Hyaloronsäure, P4HB, Polyvinylpyrolidon, Liposome, Nanopartikel, Seidenproteine und Cyclodextrine. Als weiterer Bestandteil des mit der pharmazeutisch aktiven Substanz aufgetragenen Trägers können Kontrastmittel und/oder anorganische und/oder organische Salze sowie weitere feste Zusatzstoffe vorgesehen sein. Diese dienen zur Verbesserung der mechanischen Haftung auf der Oberfläche des Ballons und/oder zur Verbesserung der Abgabe der pharmazeutisch aktiven Substanz an die Gefäßwand und/oder zur Verbesserung der Aufnahmefähigkeit der Gefäßwand für die pharmazeutisch aktive Substanz.

Bei der Verwendung eines Trägers für die pharmazeutisch aktive Substanz, das ein auszutreibendes Lösungsmittel enthält, wird das erfindungsgemäße Verfahren vorzugsweise derart abgewandelt, dass nach dem Auftragen des Trägers mit Lösungsmittel und pharmazeutisch aktiver Substanz der Ballon gefaltet wird und das nach dem Auftragen außen liegende, überschüssige Material entfernt wird. Anschließend wird der Ballon aufgeblasen (dilatiert) und das Lösungsmittel, beispielsweise durch eine Wärmebehandlung, ausgetrieben. Danach ist die pharmazeutisch aktive Substanz, die ggf. in dem Träger weitere, oben beschriebene Zusatzstoffe enthält, fest auf der Oberfläche des Ballons angeordnet. Anschließend kann der Ballon wieder gefaltet werden und die pharmazeutisch aktive Substanz ist unter den Flügeln des Ballons angeordnet, wobei hierfür der Ballon durch Verwendung eines erfindungsgemäßen definiert faltenden Ballon an den gleichen Stellen wie bei der ersten Faltung zusammen gefaltet wird, so dass die mindestens eine pharmazeutisch aktive Substanz zuverlässig unter den Flügeln des Ballons liegt.

Zur Verbesserung der Gleichmäßigkeit der aufgebrachten pharmazeutisch aktiven Substanz wird diese in einem vorteilhaften Ausführungsbeispiel des erfindungsgemäßen Verfahrens dann auf dem Ballon aufgebracht, wenn sich der Ballon im aufgeblasenen (dilatierten) Zustand befindet.

In einem weiteren bevorzugten Ausführungsbeispiel eines erfindungsgemäßen Herstellungsverfahrens für einen Katheter wird der Innenschaft und der Außenschaft des Katheters vor dem zueinander verdreht und/oder verschoben, wobei der Ballon vor dem Verdrehen und/oder Verschieben bereits mit Innenschaft und Außenschaft verbunden ist. Der verdrehte und/oder verschobene Zustand ist bei diesem Ausführungsbeispiel der Normalzustand des Instruments. Beim Dilatieren speichern Innenschaft und Außenschaft die Torsionsenergie und begünstigen somit das spiralige Rückfalten des Ballons beim Deflatieren.

Schließlich wird die letztgenannte obige Aufgabenstellung durch ein Systems bestehend aus einem Katheter mit einem Ballon und einer intraluminalen Endoprothese gelöst, wobei zunächst der Katheter nach einem der oben beschriebenen Verfahren hergestellt, anschließend an dem Ballon mindestens ein Flügel gebildet, dann dieser mindestens eine Flügel an den Innenschaft eng angelegt wird und danach die intraluminale Endoprothese auf dem gefalteten Ballon derart fest angeordnet wird, dass sie diesen mindestens teilweise umgibt.

Das erfindungsgemäße Herstellungsverfahren des Systems ist kostengünstig und leicht durchführbar und ergibt ein System, das die gewünschte lokale Behandlung des Körperhohlraums ermöglicht.

Weiter vereinfacht wird das Herstellungsverfahren, wenn die intraluminale Endoprothese mittels Aufcrimpen auf dem Ballon angeordnet wird.

In einem bevorzugten Ausführungsbeispiel werden die distalen und proximalen Enden des Ballons beim Aufbringen mindestens einer pharmazeutisch aktiven Substanz ausgespart, vorzugsweise durch Abdecken während des Aufbringens der Substanz. Eine unkontrollierte Freigabe der mindestens einen pharmazeutisch aktiven Substanz aus den Bereichen des proximalen oder distalen Endes des Ballons, die möglicherweise nicht vollständig von der Endoprothese umgeben sind, wird hierdurch verhindert.

Wie bereits oben erläutert, ist es von Vorteil, wenn die intraluminale Endoprothese vor der Anordnung der intraluminalen Endoprothese auf dem Ballon luminal mit einer leicht abwaschbaren Beschichtung, vorzugsweise mit einer oder mehrerer der Substanzen aus der Gruppe Zucker, vorzugsweise Polysaccharide, Glykane, Glucose, Glycogen, Amylose, Amylopektin, Chitin, Callose und Zellulose, und Fette, vorzugsweise Cholesterin, Cholesterol, Palmöl, partiell hydrierte Sojaöle und gesättigte Öle, versehen wird.

Weitere Ziele, Merkmale, Vorteile und Anwendungsmöglichkeiten der Erfindung ergeben sich aus der nachfolgenden Beschreibung von Ausführungsbeispielen anhand der Figuren. Dabei bilden alle beschriebenen und/oder bildlich dargestellten Merkmale für sich oder in beliebiger Kombination den Gegenstand der vorliegenden Erfindung, auch unabhängig von ihrer Zusammenfassung in den einzelnen Ansprüchen oder deren Rückbeziehung.

Es zeigen schematisch:
- Fig. 1: ein erstes Ausführungsbeispiel eines Katheters in einer Ansicht von der Seite, wobei der Ballon im dilatierten Zu- stand gezeigt ist,
- Fig. 2a, b und c: Querschnitte weiterer Ausführungsbeispiele eines Katheters mit einem, zwei und drei Flügeln im gefalteten Zu- stand,
- Fig. 3a, b und c: die Ausführungsbeispiele gemäß Fig. 2a, b und c in dem Zustand, in dem die Flügel eng an dem Innenschaft anliegen, dargestellt als Querschnitte,
- Fig.4: ein fünftes Ausführungsbeispiel eines erfindungsgemäßen Kathe- ters in einer Ansicht von der Seite bei offenem oder dilatiertem Bal- lon (ohne Druck), wobei der Ballon unterbrochene umlaufende Ver- tiefungen aufweist,
- Fig. 5: ein vergrößerter Ausschnitt der Ballonoberfläche des fünften Aus- führungsbeispiels,
- Fig. 6: das fünfte Ausführungsbeispiel während der Herstellung desselben in einer Ansicht von der Seite bei offenem oder dilatiertem Ballon,
- Fig. 7: einen Querschnitt eines sechsten Ausführungsbeispiels eines erfin- dungsgemäßen Katheters mit drei Flügeln und einer pharmazeu- tisch aktiven Substanz,
- Fig. 8a, b und c: Querschnitte dreier weiterer Ausführungsbeispiele eines erfin- dungsgemäßen Katheters mit je mindestens einem Self-X-Gerüst bei dilatiertem Ballon,
- Fig. 9a, b und c: Querschnitte der Ausführungsbeispiele gemäß Fig. 8a, b und c im teilweise deflatierten Zustand,
- Fig. 10a, b und c: Querschnitte der Ausführungsbeispiele gemäß Fig. 8a, b und c im gegenüber Fig. 9a, b und c weiter deflatierten Zustand,
- Fig. 11a, b und c: Querschnitte dreier weiterer Ausführungsbeispiele eines erfin- dungsgemäßen Katheters mit unterschiedlichen Materialien in oder an der Ballonoberfläche bei dilatiertem Ballon,
- Fig. 12a, b und c: Querschnitte der Ausführungsbeispiele gemäß Fig. 11a, b und c im teilweise deflatierten Zustand und
- Fig. 13a, b und c: Querschnitte der Ausführungsbeispiele gemäß Fig. 11a, b und c im gegenüber Fig. 9a, b und c weiter deflatierten Zustand.

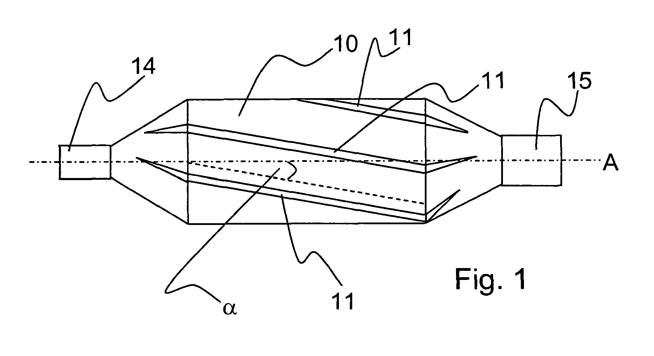

Das in Figur 1 dargestellte erste Ausführungsbeispiel eines Katheters zeigt den Ballon 10 des Katheters im dilatierten Zustand (ohne Druck). Der Ballon 10 weist mehrere jeweils über den größten Teil ihrer Länge parallel verlaufende Faltelemente (im Folgenden auch Faltlinien genannt) 11 auf, die unter einem Winkel α zur Katheterachse A (α ≠ 0°) auf der Oberfläche des Ballons 10 verlaufen. Die Faltlinien 11 erstrecken sich im Wesentlichen entlang der Längsrichtung, die die Katheterachse A verkörpert. Jede Faltlinie 11 ist als Vertiefung mit einer bestimmten Breite in der Ballonmembran ausgebildet, alternativ kann die Faltlinie 11 auch als Erhöhung mit einer bestimmten Breite oder Wandstärkensprung bzw. Wandstärkenänderung ausgebildet sein. Hierdurch wird erreicht, dass der Ballon 10 im Bereich der Faltlinie 11 eine höhere Steifigkeit als die übrigen Bereiche des Ballons aufweist. Am vorderen, distalen Ende ist der Ballon 10 mit dem Innenschaft 14 verbunden, während er am hinteren, proximalen Ende an dem Außenschaft 15 befestigt ist. Vorzugsweise ist der Ballon 10 mit Innenschaft 14 bzw. Außenschaft 15 verschweißt.

Im Bereich ihrer distalen und proximalen Enden verlaufen die Faltlinien 11 nicht parallel und gehen allmählich in den angrenzenden Bereich des Ballons 10 über. Dies bedeutet, dass beispielsweise im Falle einer als Vertiefung ausgebildeten Faltlinie 11 deren Tiefe im Bereich ihrer Enden langsam abnimmt, bis die Faltlinie 11 die Tiefe Null annimmt. Gleichzeitig nimmt die Breite der Faltlinien 11 im Bereich ihrer Enden ab.

Die Breite und Höhe der Erhebungen bzw. Vertiefungen hängt vom Durchmesser des Ballons 10, dem verwendeten Ballonmaterial sowie der Wandstärke der Ballonmembran ab. Die Breite der Erhebungen oder Vertiefungen ist mindestens doppelt so groß wie die Wandstärke der Ballonmembran. Vorzugsweise wird das Ballonmaterial derart thermogeformt, dass die Rillen nichts von der ursprünglichen Wandstärke des Ballons abnehmen, sondern dass die Kontur sich ändert (vergleichbar mit einer Wellblechform).

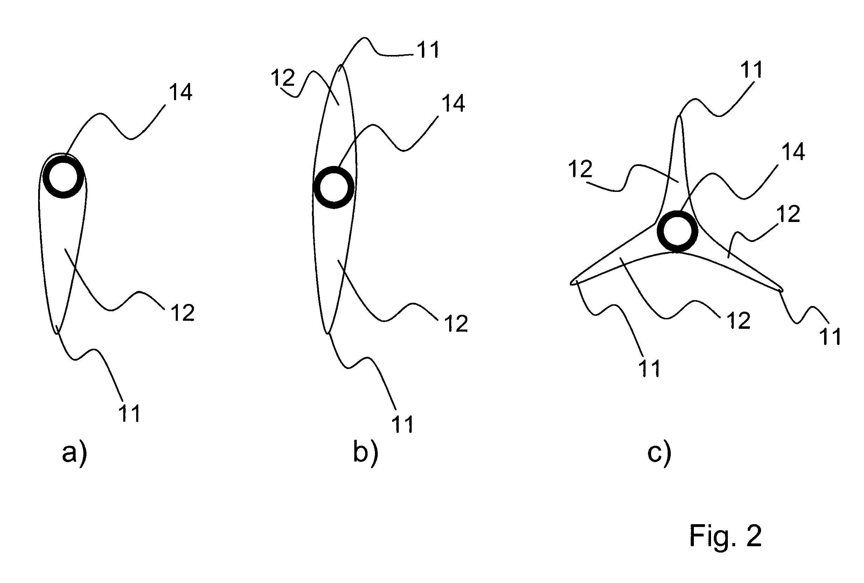

In dem in Figur 2a, b und c gezeigten Querschnitten des ersten Ausführungsbeispiels ist erkennbar, dass die Faltlinien 11 jeweils am vorderen Ende des Flügels 12 angeordnet sind, und damit jeweils im Bereich mit dem kleinsten Biegeradius. Ein Minimum im Biegeradius der Flügel 12 liegt etwa dort, wo das vordere Ende der Flügel 12 angeordnet ist, d.h. der Flügel 12 wird entlang der Faltlinie 11 gefaltet. Alternativ oder zusätzlich kann eine Faltlinie dort vorgesehen werden, wo, wie bei den Varianten in Figuren 2b und c mit zwei bzw. drei Flügeln 12 dargestellt ist, der eine Flügel 12 an den anderen Flügel angrenzt. In den Figuren 2a, b und c ist außerdem der innen liegende Innenschaft 14 des Katheters gezeigt, der von dem Ballon 10 umgeben wird.

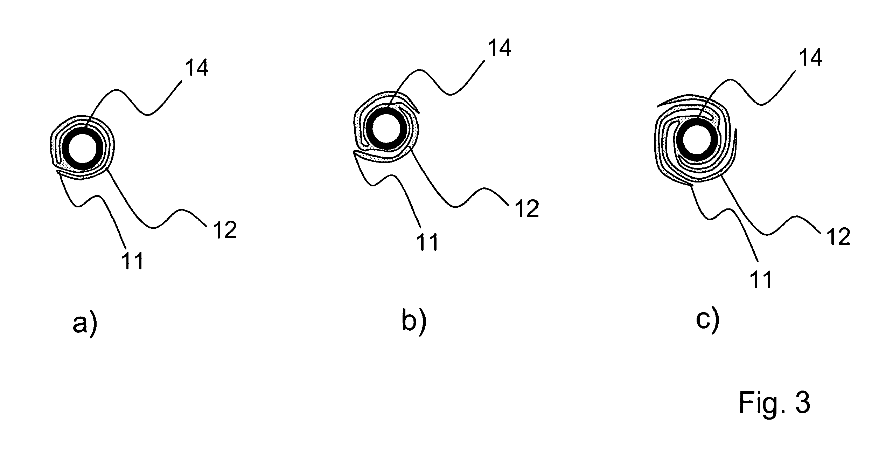

In den Figuren 3a, b und c sind die Katheter der Figuren 2a, b und c noch einmal in dem Zustand gezeigt, in dem die Flügel 12 eng an dem Innenschaft 14 anliegen. Dies wird dadurch erreicht, dass die Flügel 12 um den Innenschaft gewickelt und in dieser Stellung geprägt werden.

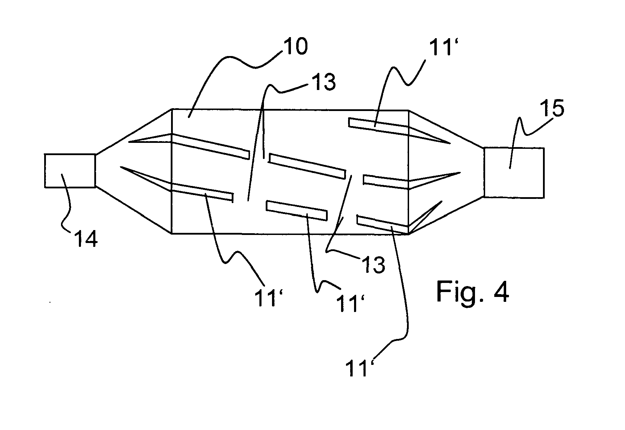

Das in Figur 4 dargestellte fünfte Ausführungsbeispiel eines erfindungsgemäßen Katheters weist Faltelemente oder Faltlinien 11' in Form von Vertiefungen auf, bei denen über ihre gesamte Länge (d.h. in Längsrichtung) mehrere Unterbrechungen 13 vorgesehen sind. Denkbar ist außerdem, lediglich eine Unterbrechung 13 entlang einer Faltlinie 11' vorzusehen.

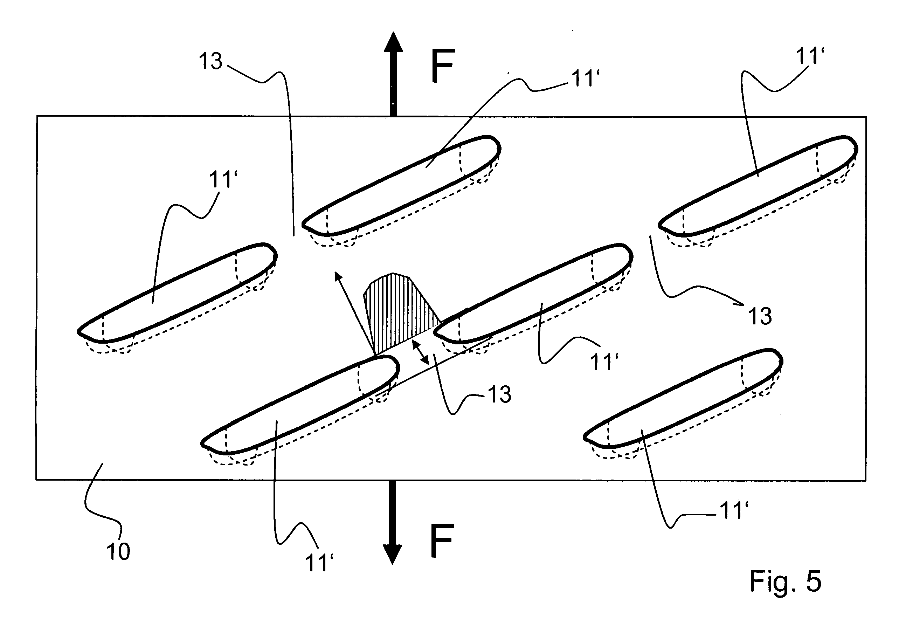

In dem in Figur 5 dargestellten Ausschnitt des Ballons 10 ist zu erkennen, dass im Bereich (Unterbrechungen, Stege) 13 zwischen den Vertiefungen entlang der als Vertiefungen ausgebildeten Faltlinien 11' bei Zugkräften F eine lokal erhöhte Zugbelastung entsteht (dargestellt als schraffierter Bereich). Hierdurch werden die Vertiefungen entlang der Faltlinien 11' beim Entlasten des Ballons wieder aufgestellt, so dass die Faltenbildung entlang der Faltlinien 11' unterstützt wird.

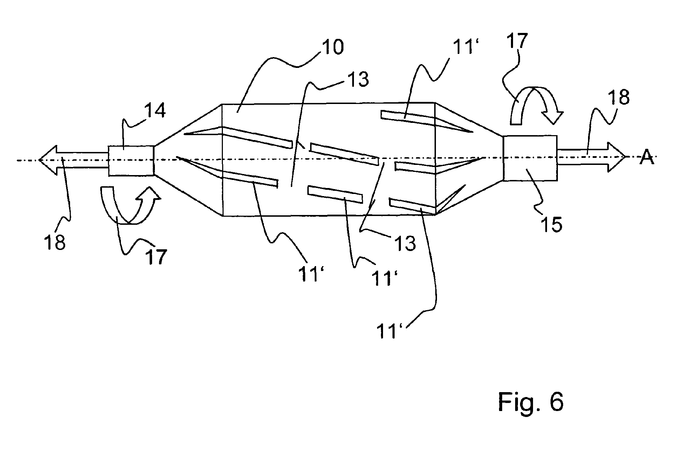

In Figur 6 ist schließlich die Herstellung des in den Figuren 4 und 5 dargestellten Ausführungsbeispiels gezeigt. Nach dem Befestigen des Ballons 10 am Innenschaft 14 und am Außenschaft 15 werden diese zueinander verdreht (verdrillt, Pfeile 17). Außerdem werden Innenschaft 14 und Außenschaft 15 in Richtung der Längsachse A voneinander weg bewegt (verschoben, vgl. Pfeile 18). Innenschaft 14 und Außenschaft 15 werden so verdrillt und verschoben aneinander befestigt. Ein derartiger Ballon 10, der im Grundzustand verdrillt und verschoben auf dem Katheter montiert ist, übt beim Dilatieren eine Torsionskraft auf den Katheter aus. Diese Torsionskraft wird im Innenschaft 14 und im Außenschaft 15 gespeichert und wirkt beim Deflatieren auf die Ballonenden. Ein solcher Ballon 10 kann mit umlaufenden Faltlinien 11' besser entlang der Faltlinien 11' zurückfalten.

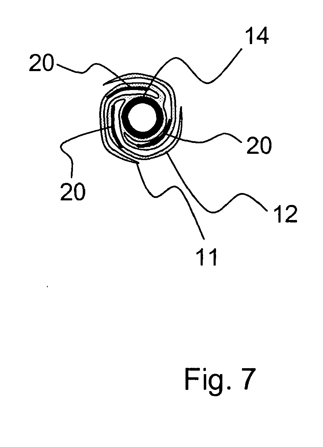

Figur 7 zeigt ein sechstes Ausführungsbeispiel eines erfindungsgemäßen Katheters, bei dem unter jedem Flügel 12 eine pharmazeutisch aktive Substanz 20 angeordnet ist. Diese pharmazeutisch aktive Substanz 20 ist vorzugsweise in oder auf einem Träger angeordnet, der fest auf der Ballonoberfläche haftet, die in den Flügelzwischenräumen zu liegen kommt.

Es soll an dieser Stelle darauf hingewiesen werden, dass die Figuren 3a, b und c sowie Figur 7 insofern eine Schematisierung enthalten, dass Teile des Flügels 12 gegenüber der Realität von den übrigen Bereichen dem jeweiligen Flügel12 abgehoben dargestellt sind. Bei der realen Faltung des Ballons 10 liegen die innen in Richtung Innenschaft 14 angeordneten Ballonbereiche jedes Flügels 12 eng an den darunter liegenden Bereichen der Flügel 12 an, so dass beim Ein- und Ausführen des Katheters lediglich kleine Kräfte (u.a. Pullbackkräfte) aufgewendet werden müssen.

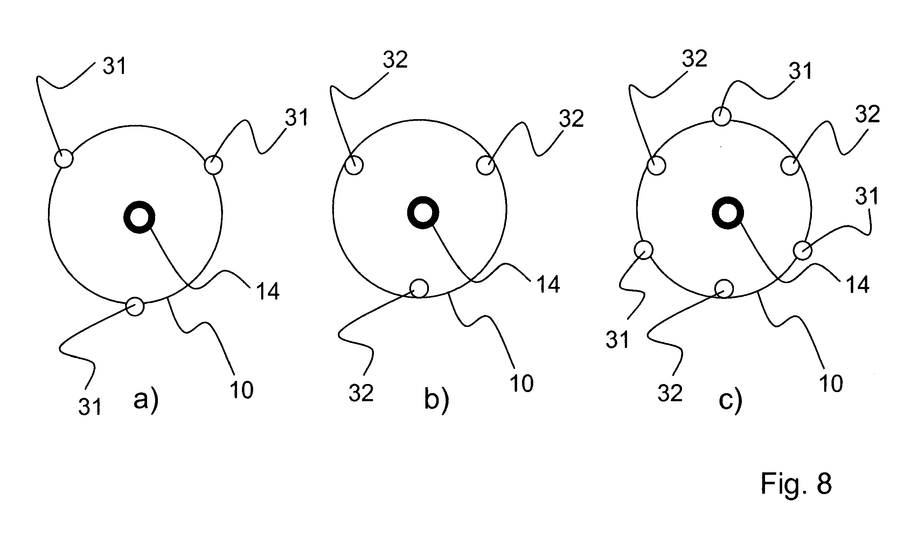

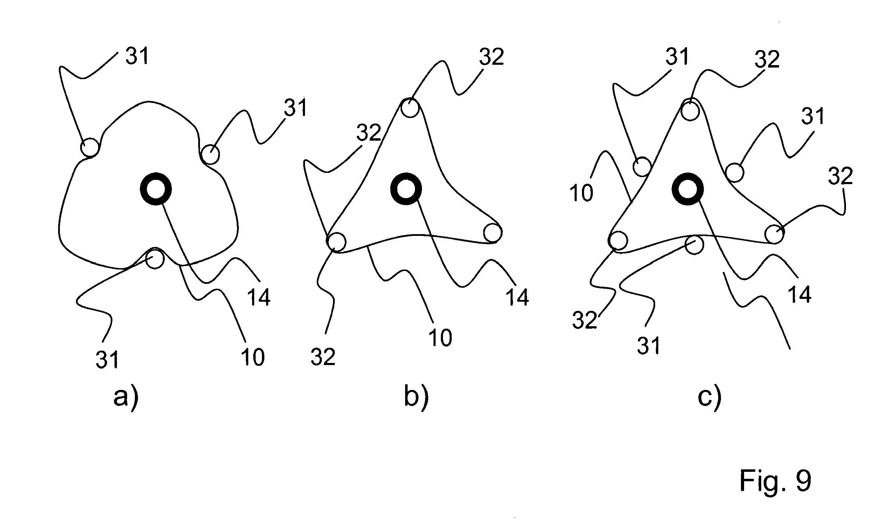

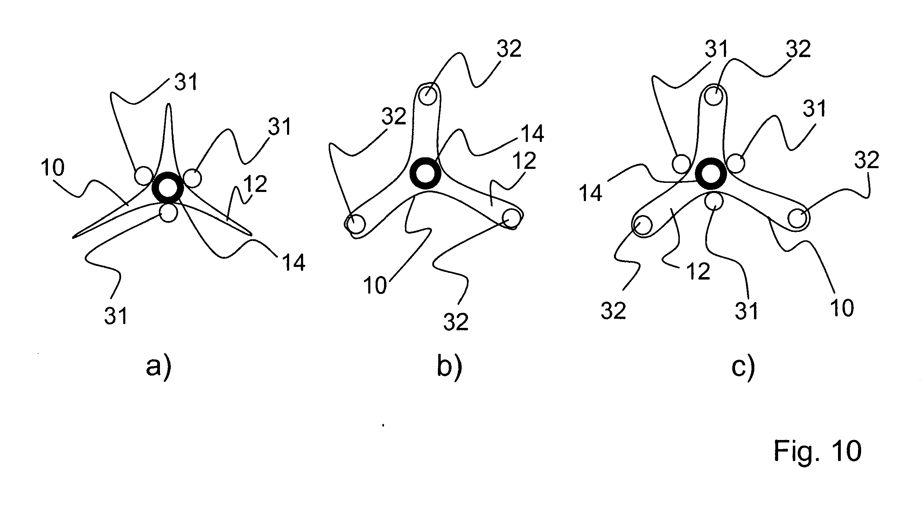

Figur 8 zeigt weitere Ausführungsbeispiele eines erfindungsgemäßen Katheters, bei denen ein Self-X-Gerüst vorgesehen ist. Das Self-X-Gerüst weist in dem in Figur 8a dargestellten Ausführungsbeispiel Längsstreben 31 als Faltelemente auf, die in Längsrichtung auf der äußeren Oberfläche des Ballons 10 verlaufen. Der in Figur 8b dargestellte Katheter weist auf der inneren Oberfläche des Ballons Längsstreben 32 als Faltelemente auf. Bei der in Figur 8c gezeigten Variante sind sowohl auf der äußeren Oberfläche des Ballons als auch auf der inneren Oberfläche des Ballons Längsstreben 31 bzw. 32 vorgesehen. Figur 9 zeigt das Verhalten der Ballonmembran beim Deflatieren der in Figur 8 dargestellten Kathetervarianten. Die auf der äußeren Oberfläche des Ballons angeordneten Längsstreben 31 in den Figuren 8a, 9a bzw. 8c, 9c entspannen sich dadurch, dass sie sich mit der darunter liegenden Ballonmembran in Richtung des Innenschafts 14 bewegen. Demgegenüber behalten die an der inneren Ballonoberfläche angeordneten Längsstreben 32 ihre Position (vgl. Figuren 9b und 9c). Durch diese, von dem Self-X-Gerüst verursachte Bewegung der Ballonmembran bildet der Ballon die entsprechende Anzahl von Flügeln 12 (siehe Figur 10) aus, wobei die Längsstreben 31 in den Minima des Biegeradius im Bereich des Übergangs zwischen zwei Flügeln 12 und die Längsstreben 32 in den Minima des Biegeradius an den vorderen Enden der Flügel 12 angeordnet sind.

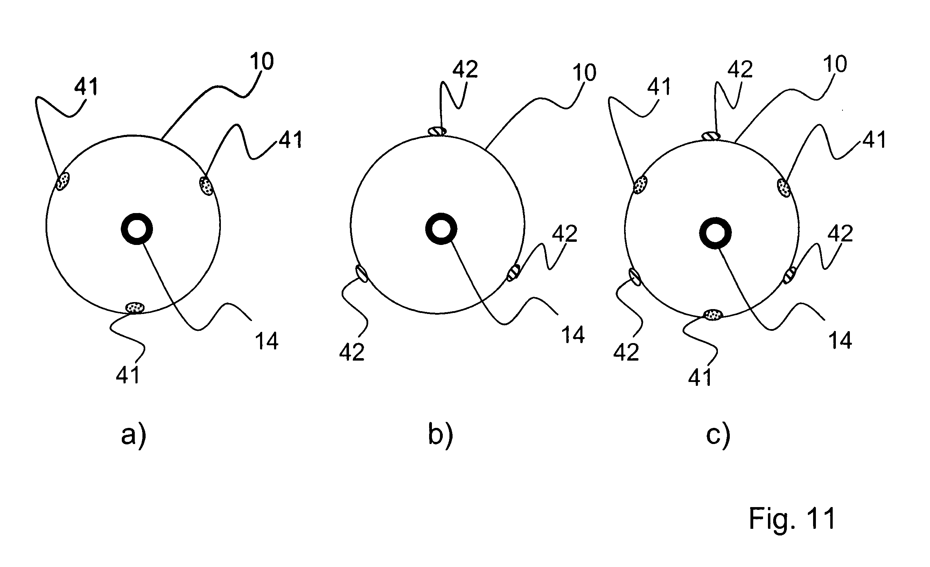

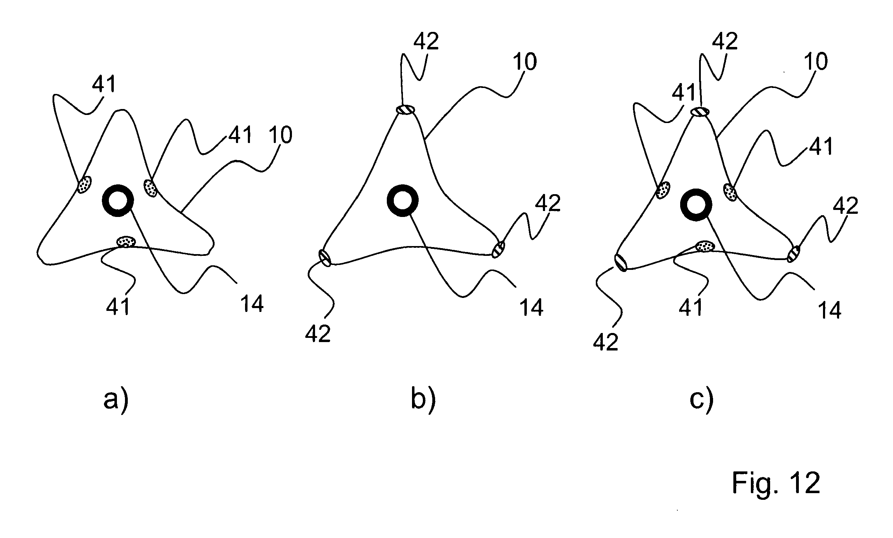

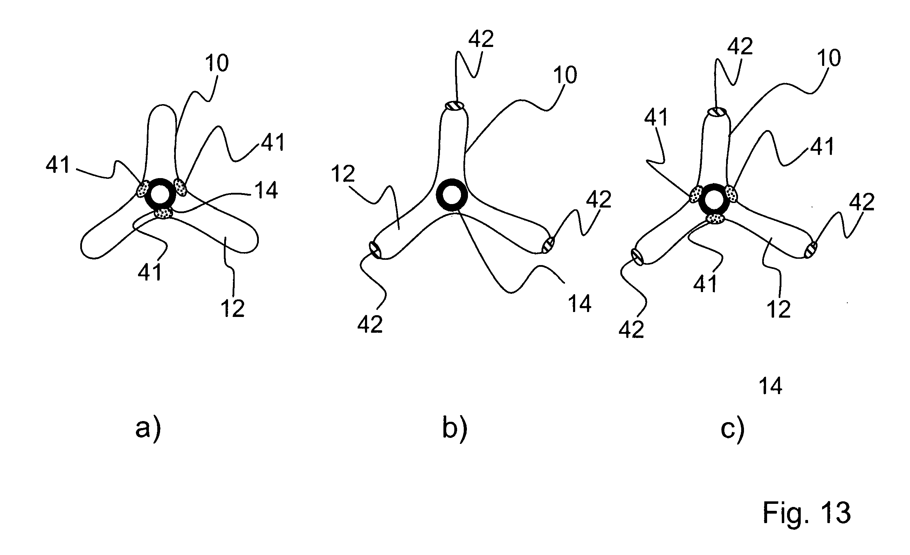

Zu den Figuren 8, 9 und 10 sei an dieser Stelle erläutert, dass jede der Teilfiguren a, b und c der genannten Figuren ein Ausführungsbeispiel eines erfindungsgemäßen Katheters in unterschiedlichen Stadien des Deflatierens zeigt, z.B stellen. die Figuren 8a, 9a und 10a das gleiche Ausführungsbeispiel in unterschiedlichen Stadien des Deflatierens dar. Gleiches gilt auch für die in den Figuren 11, 12 und 13 dargestellten Ausführungsbeispiele.

Die Figuren 11, 12 und 13 zeigen einen Ballon, in dem Bereiche (Stege) aus von dem Ballonmaterial verschiedenem Werkstoff als Faltelemente wirken. In den Figuren 11a, 12a und 13a ist ein Ballon 10 aus dem Material PEBAX dargestellt, der auf der Innenseite der Ballonoberfläche Stege 41 aus dem Material PA12 aufweist, die in Längsrichtung des Ballons verlaufen. Beim Inflatieren widerstehen die PA12-Stege 41 stärker dem Balloninnendruck und speichern so mehr elastische Energie als die Ballonwand aus PEBAX. Beim Deflatieren reicht der resultierende Formunterschied, so dass die Stege 41 in den Falten, die den Übergang zwischen zwei Flügeln 12 bilden, angeordnet werden.

In den Figuren 11b, 12b und 13b dargestellten Ausführungsbeispiel ziehen sich die außen angebrachten Stege beim Entlasten weniger stark zusammen als die umgebende Ballonwand, wenn sie z.B. weniger eingefrorene Zugspannung vom Thermoformvorgang gespeichert haben. Hier wird als Ballonmaterial PA12 verwendet, während die Stege 42, die außen auf der Ballonoberfläche angeordnet sind und in Längsrichtung des Katheters verlaufen, z.B. aus PEBAX bestehen. Die Faltelemente 42 werden beim Deflatieren, da sie unter geringerer Zugspannung als die Ballonwand stehen, in der Falte am vorderen Ende der Flügel 12 angeordnet.

Die Figuren 11c, 12c und 13c zeigen ein Ausführungsbeispiel, das die in 11a, 12a und 13a bzw. 11 b, 12b und 13b dargestellten Ausführungsbeispiele kombiniert, wobei als Ballonmaterial PEBAX verwendet wird. Hierbei stellt Steg 42 einen Bereich erhöhter Wandstärke dar. Aufgrund der beim Thermoformvorgang geringeren Dehnung der Außenmembran im Vergleich zur Innenmembran ist ein Steg, der auf der Außenoberfläche angebracht ist direkt nach dem Formen grundsätzlich spannungsärmer als die Innenmembran bzw. Ballonmembran.

Ein erfindungsgemäßer Katheter kann mittels des folgenden Verfahrens hergestellt werden:
Zunächst wird der Ballon 10 beispielsweise mittels Blasformen hergestellt, wobei die Blasform dort, wo die Faltlinien 11 vorgesehen werden sollen, Erhebungen aufweist, so dass in dem Ballon 10 an der entsprechenden Stelle Vertiefungen entstehen. Alternativ kann ein Ballon 10 mit Stegen mittels Spritzblasen hergestellt werden. Anschließend wird der Ballon 10 mit den Faltlinien 11, die vorzugsweise in einem Winkel α zu der Ballonachse A verlaufen, mit dem Kathetergrundkörper, d.h. im Einzelnen mit dem Innenschaft 14 und dem Außenschaft 15 verbunden.

Das in den Figuren 4 bis 6 dargestellte Ausführungsbeispiel mit einem verdrillten Ballon, der in seinem verdrillten Zustand dauerhaft thermisch fixiert wurde, stellt eine weitere vorteilhafte Alternative dar, einen selbstrückfaltenden, d.h. einen definiert rückfaltenden, Ballon mit gleichmäßigem Biegemoment herzustellen.

In einem weiteren Ausführungsbeispiel des erfindungsgemäßen Verfahrens kann der Ballon auch nachträglich, d.h. nach dem Verbinden mit Innen- und Außenschaft, beispielsweise im dilatierten Zustand, mit Faltlinien 11 versehen werden, indem die Ballonmembran z.B. mittels eines Lasers lokal thermisch behandelt wird. Der Laser kann beispielsweise so eingesetzt werden, dass der Fokus des Lasers entlang der einzubringenden Faltlinien 11 geführt und in diesem Bereich die obere Schicht der Ballonmembran thermisch entfernt wird. Alternativ kann ein Lösungsmittel eingesetzt werden, das die Bereiche entlang der einzubringenden Faltlinien 11 benetzt und dort das Gefüge der Ballonmembran verändert. Hierdurch wird jeweils die Steifigkeit der Ballonmembran im thermisch veränderten oder durch das Lösungsmittel behandelten Bereich gegenüber der Umgebung verändert.

### Bezugszeichenliste:

- 10: Ballon
- 11, 11': Faltlinie
- 12: Flügel
- 13: Unterbrechung
- 14: Innenschaft
- 15: Außenschaft
- 16: äußere Fläche der Flügel 12
- 17: Pfeil (Torsion, Verdrehung)
- 18: Pfeil (Verschiebung)
- 20: pharmazeutisch aktive Substanz
- 31, 32: Längsstrebe eines Self-X-Gerüsts
- 41, 42: in Längsrichtung verlaufender Steg
- α: Winkel der Faltlinie 11 zur Ballonachse A
- A: Ballonachse
- F: Zugkraft

## Patentansprüche

1. Katheter mit einem Ballon (10), der im nicht dilatierten Zustand mindestens einen Flügel (12), und am und/oder im Ballonmaterial (10) für jeden Flügel (12) mindestens ein im Wesentlichen in Längsrichtung verlaufendes Faltelement (11, 11', 31, 32, 41, 42) aufweist, wobei das Faltelement beim Falten des Ballons (10) in einem der in Längsrichtung verlaufenden Bereiche des Flügels (12) mit einem Minimum im Biegeradius angeordnet ist, **dadurch gekennzeichnet, dass** das mindestens eine Faltelement (11') entlang der Längsrichtung Unterbrechungen (13) aufweist.

2. Katheter nach Anspruch 1, **dadurch gekennzeichnet, dass** das mindestens eine Faltelement (11, 11', 31, 32, 41, 42) einen Bereich des Ballons (10) ausbildet, der verglichen mit den übrigen Bereichen des Ballons (10) eine veränderte Steifigkeit, vorzugsweise eine niedrigere Steifigkeit, aufweist und/oder dass das mindestens eine Faltelement (31, 32, 41, 42) einen Bereich des Ballons (10) ausbildet, der eine von den übrigen Bereichen des Ballons (10) verschiedene Materialzusammensetzung aufweist und/oder dass das mindestens eine Faltelement (11, 11') in einem fest vorgegebenen Winkel (α) zur Ballonachse (A) verläuft.

3. Katheter nach Anspruch 2, **dadurch gekennzeichnet, dass** der Ballon (10) in dem Bereich des Faltelements (11, 11') Vertiefungen oder Erhebungen oder mindestens einen Wandstärkensprung aufweist.

4. Katheter nach Anspruch 1, **dadurch gekennzeichnet, dass** die Faltelemente (11) des Ballons (10) durch Längsstreben (31, 32) ausgebildet werden, die ein Gerüst bilden, welches an der Innenseite und/oder der Außenseite des Ballons (10) angeordnet ist und das den Ballon (10) an definierten Stellen stützt.

5. Katheter nach einem der vorhergehenden Ansprüche, **dadurch gekennzeichnet, dass** im nicht dilatierten Zustand mindestens eine pharmazeutisch aktive Substanz (20), vorzugsweise enthaltend Taxole und/oder Taxane, besonders bevorzugt Paclitaxel oder Sirolimus, und/oder vorzugsweise enthaltend mindestens einen hyperplastischen Wirkstoff mit einem Verteilungskoeffizienten zwischen Butanol und Wasser von ≥ 0,5, zumindest teilweise unter dem mindestens einen Flügel (12) des Ballons (10) angeordnet ist und/oder dass die mindestens eine pharmazeutisch aktive Substanz (20) auf einem Träger oder mehreren Trägern angeordnet ist, die unter einem Flügel (12) angeordnet ist oder mehreren Flügeln (12) des Ballons (10) angeordnet sind.

6. Katheter nach Anspruch 5, **dadurch gekennzeichnet, dass** der mindestens eine Flügel (12) des Ballons (10) mittels der mindestens einen pharmazeutisch aktiven Substanz (20) verklebt ist und/oder dass die mindestens eine pharmazeutisch aktive Substanz (20) mittels Tauchen, Sprühen, Bestreichen oder Pressen auf dem Ballon (10), ggf. eingebettet in einen Träger, aufgebracht ist.

7. System zum Einbringen einer intraluminalen Endoprothese, vorzugsweise eines Stents, in einen Körperhohlraum bestehend aus der intraluminalen Endoprothese und einem Katheter nach einem der vorhergehenden Ansprüche, **dadurch gekennzeichnet, dass** die intraluminale Endoprothese auf dem gefalteten Ballon (10) derart fest angeordnet ist, dass sie diesen mindestens teilweise umgibt, wobei die intraluminale Endoprothese auf den Ballon (10) aufgecrimpt ist.

8. System nach Anspruch 7, **dadurch gekennzeichnet, dass** die intraluminalen Endoprothese als biodegradierbarer Stent, vorzugsweise als AMS-Stent, ausgebildet ist.

9. System nach Anspruch 7 oder 8, **dadurch gekennzeichnet, dass** die intraluminale Endoprothese die darunter liegenden äußeren Flächen (16) des gefalteten Ballons (10) im nicht dilatierten Zustand vollständig bedeckt und/oder dass die intraluminale Endoprothese luminal mit einer leicht lösbaren Beschichtung versehen ist, vorzugsweise mit einer oder mehrerer der Substanzen aus der Gruppe Zucker, vorzugsweise Polysaccharide, Glykane, Glucose, Glycogen, Amylose, Amylopektin, Chitin, Callose und Zellulose, und Fette, vorzugsweise Cholesterin, Cholesterol, Palmöl, partiell hydrierte Sojaöle und gesättigte Öle, versehen wird.

10. Verfahren zur Herstellung eines Katheters nach einem der Ansprüche 1 bis 6, **dadurch gekennzeichnet, dass** zunächst der Ballon (10) mit mindestens einem Faltelement (11, 11', 31, 32, 41, 42) versehen wird, danach der Kathetergrundkörper mit Innenschaft (14) und Außenschaft (15) bereit gestellt wird und anschließend der Ballon (10) mit Innenschaft (14) und Außenschaft (15) verbunden wird und/oder dass der Ballon (10) außen und/oder innen mit einem aus Längsstreben (31, 32) bestehenden Gerüst verbunden wird, welches den Ballon (10) in bestimmten Bereichen stützt.

11. Verfahren nach Anspruch 10, **dadurch gekennzeichnet, dass** der Ballon (10) mittels Blasformen hergestellt wird, wobei die Blasform dort, wo das mindestens eine Faltelement (11, 11') entstehen soll, mit einer Vertiefung oder einer Erhebung versehen ist oder dass der Ballon (10) mittels Spritzblasen derart hergestellt wird, dass dort, wo das mindestens eine Faltelement (11, 11') entstehen soll, mindestens ein definierter Wandstärkensprung erzeugt wird.

12. Verfahren nach Anspruche 10 oder 11, **dadurch gekennzeichnet, dass** der Ballon (10) im Bereich des mindestens einen Faltelements (11, 11', 31, 32, 41, 42), vorzugsweise vor einem Aufbringen einer pharmazeutisch aktiven Substanz (20) auf die Oberfläche des Ballons (10), lokal thermisch behandelt wird und/oder dass der Ballon (10), vorzugsweise vor dem Aufbringen einer pharmazeutisch aktiven Substanz (20), im Bereich des mindestens einen Faltelements (11, 11', 31, 32, 41, 42) mittels eines Lösungsmittels behandelt wird und/oder dass der Ballon (10), vorzugsweise vor einem Aufbringen einer pharmazeutisch aktiven Substanz (20), im Bereich außerhalb des mindestens einen Faltelements (11, 11', 31, 32, 41, 42) mit einem zusätzlich verstärkenden Material versehen wird.

13. Verfahren nach einem der Ansprüche 10 bis 12, **dadurch gekennzeichnet, dass** auf den Ballon (10) des Katheters vor dem Falten zunächst mindestens eine pharmazeutisch aktive Substanz (20), ggf. eingebettet in einen Träger, auf und/oder in die äußere Oberfläche des Ballons (10) ein- oder aufgebracht wird, vorzugsweise mittels Tauchen, Sprühen, Bestreichen oder Pressen, wobei die pharmazeutisch aktive Substanz vorzugsweise Taxole und/oder Taxane, besonders bevorzugt Paclitaxel oder Sirolimus, und/oder vorzugsweise mindestens einen hyperplastischen Wirkstoff mit einem Verteilungskoeffizienten zwischen Butanol und Wasser von ≥ 0,5,enthält, wobei die pharmazeutisch aktive Substanz eingebettet in einen Träger auf den Ballon aufgebracht wird, wobei in dem Träger ggf. ein Kontrastmittel und/oder ein anorganisches Salz und/oder ein organisches Salze und/oder mindestens ein weiterer Zusatzstoff vorgesehen ist, der beispielsweise zur Verbesserung der mechanischen Haftung auf der Ballonoberfläche und/oder zur Verbesserung der Abgabe der pharmazeutisch aktiven Substanz an die Gefäßwand und/oder zur mazeutisch aktiven Substanz an die Gefäßwand und/oder zur Verbesserung der Aufnahmefähigkeit der Gefäßwand dient.

14. Verfahren nach Anspruch 13, **dadurch gekennzeichnet, dass** die überschüssige mindestens eine pharmazeutisch aktive Substanz (20) und ggf. das Trägermaterial, ggf. mit mindestens einem weiteren Zusatzstoff, auf den nach dem Falten außen liegenden Oberflächen (16) des Ballons (10) nach dem Faltschritt entfernt werden, vorzugsweise durch Wegwischen und/oder dass die mindestens eine pharmazeutisch aktive Substanz (20) ausgehärtet oder polymerisiert wird und/oder dass die pharmazeutisch aktive Substanz (20) dann auf dem Ballon (10) aufgebracht wird, wenn sich der Ballon (10) im dilatierten Zustand befindet.

15. Verfahren nach einem der Ansprüche 10 bis 14, **dadurch gekennzeichnet, dass** der Innenschaft (14) und der Außenschaft (15) des Katheters vor dem Verbinden zueinander verdreht und/oder verschoben werden, wobei der Ballon (10) vor dem Verdrehen und/oder Verschieben bereits mit Innenschaft (14) und Außenschaft (15) verbunden ist.

16. Verfahren zur Herstellung eines Systems nach einem der Ansprüche 7 bis 9 bestehend aus einem Katheter mit einem Ballon (10) und einer intraluminalen Endoprothese, **dadurch gekennzeichnet, dass** zunächst der Katheter nach einem Verfahren nach einem der Ansprüche 10 bis 15 hergestellt wird, anschließend an dem Ballon (10) mindestens ein Flügel (12) gebildet, dann dieser mindestens eine Flügel (12) an den Innenschaft (14) eng angelegt wird und danach die intraluminale Endoprothese auf dem gefalteten Ballon (10) derart fest angeordnet wird, dass sie diesen mindestens teilweise umgibt, wobei die intraluminale Endoprothese mittels Aufcrimpen auf dem Ballon (10) angeordnet wird und vor der Anordnung der intraluminalen Endoprothese auf dem Ballon (10) luminal mit einer leicht abwaschbaren Beschichtung, vorzugsweise mit einer oder mehrerer der Substanzen aus der Gruppe Zucker, vorzugsweise Polysaccharide, Glykane, Glucose, Glycogen, Amylose, Amylopektin, Chitin, Callose und Zellulose, und Fette, vorzugsweise Cholesterin, Cholesterol, Palmöl, partiell hydrierte Sojaöle und gesättigte Öle, versehen wird.

## Claims

1. A catheter, comprising a balloon (10) having at least one wing (12) in the undilated state and having on and/or in the balloon material (10) at least one fold element (11, 11', 31, 32, 41, 42) running substantially in the longitudinal direction for each wing (12), wherein, when the balloon (10) is folded, the fold element is arranged in a region of the wing (12) running in the longitudinal direction with a minimum in the bending radius, **characterised in that** the at least one fold element (11') has interruptions (13) along the longitudinal direction.

2. The catheter according to Claim 1, **characterised in that** the at least one fold element (11, 11', 31, 32, 41, 42) forms a region of the balloon (10) which has an altered rigidity, preferably a lower rigidity, in comparison with the other regions of the balloon (10), and/or **in that** the at least one fold element (31, 32, 41, 42) forms a region of the balloon (10) which has a different material composition from the other regions of the balloon (10), and/or **in that** the at least one fold element (11, 11') runs at a fixedly predefined angle (α) to the balloon axis (A).

3. The catheter according to Claim 2, **characterised in that** the balloon (10) has depressions or elevations or at least one sudden change in wall thickness in the region of the fold element (11, 11').

4. The catheter according to Claim 1, **characterised in that** the fold elements (11) of the balloon (10) are formed by longitudinal struts (31, 32) which form a frame that is arranged on the inner side and/or outer side of the balloon (10) and supports the balloon (10) at defined points.

5. The catheter according to one of the preceding claims, **characterised in that**, in the undilated state at least one pharmaceutically active substance (20), preferably containing taxols and/or taxans, more preferably paclitaxel or sirolimus, and/or preferably containing at least one hyperplastic active ingredient having a distribution coefficient between butanol and water of ≥ 0.5 is arranged at least partially beneath the at least one wing (12) of the balloon (10), and/or **in that** the at least one pharmaceutically active substance (20) is arranged on one or more carriers arranged beneath a wing (12) or a number of wings (12) of the balloon (10).

6. The catheter according to Claim 5, **characterised in that** the at least one wing (12) of the balloon (10) is adhesively bonded by means of the at least one pharmaceutically active substance (20), and/or **in that** the at least one pharmaceutically active substance (20) is applied to the balloon (10), by means of dipping, spraying, painting or pressing, optionally embedded in a carrier.

7. A system for introducing an intraluminal endoprosthesis, preferably a stent, into a bodily cavity, said system consisting of the intraluminal endoprosthesis and a catheter according to one of the preceding claims, **characterised in that** the intraluminal endoprosthesis is fixedly arranged on the folded balloon (10) in such a way that the intraluminal endoprosthesis at least partially surrounds the folded balloon, wherein the intraluminal endoprosthesis is crimped onto the balloon (10).

8. The system according to Claim 7, **characterised in that** the intraluminal endoprosthesis is formed as a biodegradable stent, preferably as an AMS.

9. The system according to Claim 7 or 8, **characterised in that** the intraluminal endoprosthesis completely covers the underlying outer surfaces (16) of the folded balloon (10) in the undilated state, and/or **in that** the intraluminal endoprosthesis is coated luminally with an easily removable coating, preferably with one or more substances from the group consisting of sugars, preferably polysaccharides, glycans, glucose, glycogen, amylose, amylopectin, chitin, callose and cellulose, and fats, preferably cholesterin, cholesterol, palm oil, partially hydrogenated soy oils and saturated oils.

10. A method for producing a catheter according to one of Claims 1 to 6, **characterised in that** the balloon (10) is first provided with at least one fold element (11, 11', 31, 32, 41, 42), after which the catheter main body having an inner shaft (14) and outer shaft (15) is provided, and then the balloon (10) is connected to the inner shaft (14) and outer shaft (15), and/or **in that** the balloon (10) is connected on the outside and/or on the inside to a frame consisting of longitudinal struts (31, 32), said frame supporting the balloon (10) in specific regions.

11. The method according to Claim 10, **characterised in that** the balloon (10) is produced by blow moulding, wherein the blow mould is provided with a depression or an elevation at the location where the at least one fold element (11, 11') is to be formed, or **in that** the balloon (10) is produced by injection blow moulding in such a way that at least one defined sudden change in wall thickness is created at the location where the at least one fold element (11, 11') is to be formed.

12. The method according to Claims 10 or 11, **characterised in that** the balloon (10) is thermally treated locally in the region of the at least one fold element (11, 11', 31, 32, 41, 42), preferably before applying a pharmaceutically active substance (20) to the surface of the balloon (10), and/or **in that** the balloon (10) is treated with a solvent in the region of the at least one fold element (11, 11', 31, 32, 41, 42), preferably before applying a pharmaceutically active substance (20), and/or **in that** the balloon (10) is provided with an additionally reinforcing material in the region outside the at least one fold element (11, 11', 31, 32, 41, 42), preferably before applying a pharmaceutically active substance (20).

13. The method according to one of Claims 10 to 12, **characterised in that** at least one pharmaceutically active substance (20) is first applied onto the balloon (10) of the catheter before folding, optionally embedded in a carrier, and/or is applied into the outer surface of the balloon (10), preferably by means of dipping, spraying, painting or pressing, wherein the pharmaceutically active substance preferably contains taxols and/or taxans, more preferably paclitaxel or sirolimus, and/or preferably at least one hyperplastic active ingredient having a distribution coefficient between butanol and water of ≥ 0.5, wherein the pharmaceutically active substance is applied embedded in a carrier to the balloon, wherein a contrast medium and/or an inorganic salt and/or an organic salt and/or at least one further additive is/are optionally provided in the carrier and is/are used to improve the mechanical adhesion to the balloon surface and/or to improve the release of the pharmaceutically active substance to the vascular wall and/or to improve the uptake ability of the vascular wall.

14. The method according to Claim 13, **characterised in that** the excess at least one pharmaceutically active substance (20) and any excess carrier material, optionally with at least one further additive, on the surfaces (16) of the balloon (10) arranged on the outside after folding are removed after the folding step, preferably by being wiped away, and/or **in that** the at least one pharmaceutically active substance (20) is cured or polymerised, and/or **in that** the pharmaceutically active substance (20) is then applied to the balloon (10) when the balloon (10) is in the dilated state.

15. The method according to one of Claims 10 to 14, **characterised in that** the inner shaft (14) and the outer shaft (15) of the catheter are twisted and/or displaced relative to one another before connection, wherein the balloon (10) is connected to the inner shaft (14) and the outer shaft (15) before the twisting and/or displacement.

16. A method for producing a system according to one of Claims 7 to 9, said system consisting of a catheter with a balloon (10) and an intraluminal endoprosthesis, **characterised in that** the catheter is first produced by a method according to one of Claims 10 to 15, then at least one wing (12) is formed on the balloon (10), then said at least one wing (12) is contacted closely with the inner shaft (14) and then the intraluminal endoprosthesis is fixedly arranged on the folded balloon (10) in such a way that the intraluminal endoprosthesis at least partially surrounds the folded balloon, wherein the intraluminal endoprosthesis is arranged on the balloon (10) by means of crimping and, before being arranged on the balloon (10), is provided luminally with a coating that is easily washed off, preferably with one or more substances selected from the group consisting of sugars, preferably polysaccharides, glycans, glucose, glycogen, amylose, amylopectin, chitin, callose and cellulose, and fats, preferably cholesterin, cholesterol, palm oil, partially hydrogenated soy oils and saturated oils.

## Revendications

1. Cathéter avec un ballon (10) comportant au moins une aile (12), dans l'état non dilaté, ainsi qu'au moins un élément de pliage (11, 11', 31, 32, 41, 42) s'étendant essentiellement dans le sens longitudinal sur et/ou dans le matériau du ballon (10), pour chaque aile (12), dans lequel l'élément de pliage est agencé avec un rayon de flexion minimum, lors du pliage du ballon (10) dans l'une des zones d'aile (12) s'étendant dans le sens longitudinal, **caractérisé en ce que** l'au moins un élément de pliage (11') comporte des brèches (13) le long du sens longitudinal.

2. Cathéter selon la revendication 1, **caractérisé en ce que** l'au moins un élément de pliage (11, 11', 31, 32, 41, 42) forme une région du ballon (10) présentant une rigidité modifiée, de préférence une rigidité inférieure, par rapport aux autres régions du ballon (10), et/ou **en ce que** l'au moins un élément de pliage (11, 11', 31, 32, 41, 42) forme une région du ballon (10) présentant une composition de matériaux différente par rapport aux autres régions du ballon (10), et/ou **en ce que** l'au moins un élément de pliage (11, 11') s'étend selon un angle fixement défini (α) par rapport à l'axe (A) du ballon.

3. Cathéter selon la revendication 2, **caractérisé en ce que** le ballon (10) comporte des renfoncements ou des surélévations dans la région de l'élément de pliage (11, 11'), ou du moins une différence d'épaisseur de paroi.

4. Cathéter selon la revendication 1, **caractérisé en ce que** les éléments de pliage (11, 11', 31, 32, 41, 42) du ballon (10) sont constitués par des renforts longitudinaux (31, 32) formant une structure agencée du côté intérieur et/ou du côté extérieur du ballon (10) et soutenant le ballon (10) à des endroits prédéfinis.

5. Cathéter selon l'une des revendications précédentes, **caractérisé en ce que** dans l'état non dilaté, au moins une substance active pharmaceutique (20), contenant de préférence du taxol et/ou des taxanes, et mieux encore du paclitaxel ou du sirolimus, et/ou contenant de préférence au moins une substance active hyper-plastique, avec un coefficient de répartition entre le butanol et l'eau, ≥ à 0,5, est placée au moins partiellement sous l'au moins une aile (12) du ballon (10), et/ou **en ce que** l'au moins une substance pharmaceutique active (20) est placée sur un substrat agencé sous une aile (12) ou sur plusieurs substrats agencés sous plusieurs ailes (12) du ballon (10).

6. Cathéter selon la revendication 5, **caractérisé en ce que** l'au moins une aile (12) du ballon (10) est collée au moyen de l'au moins une substance pharmaceutique active (20), et/ou **en ce que** l'au moins une substance pharmaceutique active (20) est appliquée par immersion, vaporisation, étalage ou pressage sur le ballon (10), éventuellement par enrobage sur un substrat.

7. Système pour l'introduction d'une endoprothèse intraluminale, de préférence un stent, dans un espace creux d'un corps, constitué d'une endoprothèse intraluminale et d'un cathéter selon l'une des revendications précédentes, **caractérisé en ce que** l'endoprothèse intraluminale est agencée fixement sur le ballon plié (10), de manière à entourer au moins partiellement celui-ci, l'endoprothèse intraluminale étant sertie sur le ballon (10).

8. Système selon la revendication 7, **caractérisé en ce que** l'endoprothèse intraluminale est conçue comme un stent biodégradable, de préférence comme un stent AMS.

9. Système selon la revendication 7 ou 8, **caractérisé en ce que** dans l'état non dilaté, l'endoprothèse intraluminale recouvre entièrement la surface extérieure (16) du ballon plié (10) se trouvant en-dessous, et/ou **en ce que** l'endoprothèse intraluminale est pourvue luminalement d'un revêtement facilement soluble, contenant de préférence l'une ou de plusieurs des substances parmi le groupe comprenant le sucre, de préférence le polysaccaride, le glycane, le glucose, le glycogène, l'amylose, l'amylopectine, la chitine, la callose et la cellulose, ainsi que des graisses, de préférence le cholestérol, l'huile de palme, les huiles de soja partiellement hydrogénées et les huiles saturées.

10. Procédé pour la fabrication d'un cathéter selon l'une des revendications 1 à 6, **caractérisé en ce que** le ballon (10) est tout d'abord pourvu d'au moins un élément de pliage (11, 11',31,32, 41, 42), puis le corps de base du cathéter est préparé avec une tige intérieure (14) et une tige extérieure (15), et le ballon (10) est ensuite relié à la tige intérieure (14) et à la tige extérieure (15), et/ou **en ce que** le ballon (10) est relié, à l'extérieur et/ou à l'intérieur, à une structure constituée de renforts longitudinaux (31, 32), destinée à soutenir le ballon (10) à des endroits prédéterminés.

11. Procédé selon la revendication 10, **caractérisé en ce que** le ballon (10) est fabriqué à l'aide de moules de soufflage, dans lequel le moule de soufflage est pourvu d'un renfoncement ou d'une surélévation, à l'endroit où l'au moins un élément de pliage (11, 11') est censé apparaître, ou **en ce que** le ballon (10) est fabriqué à l'aide d'un soufflage à injection, de manière à obtenir au moins une différence d'épaisseur de paroi prédéfinie, à l'endroit où l'élément de pliage (11, 11') est censé apparaître.

12. Procédé selon les revendications 10 ou 11, **caractérisé en ce que** le ballon (10) est traité localement, de façon thermique, dans la région de l'au moins un élément de pliage (11, 11', 31, 32, 41, 42), de préférence avant une application d'une substance pharmaceutique active (20) sur la surface du ballon (10), et/ou **en ce que** le ballon (10) est traité au moyen d'un solvant, dans la région de l'au moins un élément de pliage (11, 11', 31, 32, 41, 42), de préférence avant l'application d'une substance pharmaceutique active (20), et/ou **en ce que** le ballon (10) est pourvu d'un matériau de renfort supplémentaire, dans la région externe à l'au moins un élément de pliage (11, 11', 31, 32, 41, 42), de préférence avant une application d'une substance pharmaceutique active (20).

13. Procédé selon l'une des revendications 10 à 12, **caractérisé en ce qu'**au moins une substance pharmaceutique active (20), éventuellement enrobée dans un substrat, est tout d'abord incorporée ou appliquée dans et/ou sur la surface extérieure du ballon (10), avant le pliage, de préférence par immersion, vaporisation, étalage ou pressage, dans lequel la substance pharmaceutique active (20) contient de préférence du taxol et/ou des taxanes, et mieux encore du paclitaxel ou du sirolimus, et/ou de préférence au moins une substance active hyper-plastique, avec un coefficient de répartition entre le butanol et l'eau, ≥ à 0,5, dans lequel la substance pharmaceutique active enrobée dans un substrat est appliquée sur le ballon, dans lequel est prévu un agent de contraste et/ou un sel anorganique et/ou un sel organique et/ou au moins un adjuvant supplémentaire, servant par exemple à améliorer l'adhérence mécanique sur la surface du ballon, et/ou à améliorer le dépôt mécanique de la substance pharmaceutique active sur la paroi de vaisseau, et/ou à améliorer la capacité d'assimilation de la paroi de vaisseau.

14. Procédé selon la revendication 13, **caractérisé en ce qu'**après le pliage, la substance pharmaceutique active (20) excédentaire et éventuellement le substrat, éventuellement avec au moins un adjuvant supplémentaire, est éliminée des surfaces (16) du ballon (10) situées à l'extérieur après le pliage, de préférence par essuyage, et/ou **en ce que** l'au moins une substance pharmaceutique active (20) est durcie ou polymérisée, et/ou **en ce que** la substance pharmaceutique active (20) est appliquée sur la ballon (10) lorsque le ballon (10) se trouve dans l'état dilaté.

15. Procédé selon l'une des revendications 10 à 14, **caractérisé en ce que** la tige intérieure (14) et la tige extérieure (15) du cathéter sont tournées et/ou déplacées l'une par rapport à l'autre, avant l'assemblage, le ballon (10) étant déjà relié à la tige intérieure (14) et à la tige extérieure (15) avant la rotation et/ou le déplacement.

16. Procédé pour la fabrication d'un système selon l'une des revendications 7 à 9, constitué d'un cathéter avec un ballon (10) et d'une endoprothèse intraluminale, **caractérisé en ce que** le cathéter est tout d'abord fabriqué à l'aide d'un procédé selon l'une des revendications 10 à 15, puis au moins une aile (12) est formée sur le ballon (10), puis cette au moins une aile (12) est serrée contre la tige intérieure (14), puis l'endoprothèse intraluminale est disposée fixement sur le ballon plié (10), de manière à entourer au moins partiellement celui-ci, l'endoprothèse intraluminale étant fixée sur le ballon (10) au moyen d'un sertissage, et pourvue luminalement d'un revêtement facilement soluble, contenant de préférence l'une ou de plusieurs des substances parmi le groupe comprenant le sucre, de préférence le polysaccaride, le glycane, le glucose, le glycogène, l'amylose, l'amylopectine, la chitine, la callose et la cellulose, ainsi que des graisses, de préférence le cholestérol, l'huile de palme, les huiles de soja partiellement hydrogénées et les huiles saturées, avant la mise en place de l'endoprothèse intraluminale.
